# EUROPEAN PATENT APPLICATION

(11) **EP 4 529 859 A1**
(43) Date of publication of application: **02.04.2025**
(21) Application number: 23203895.0
(22) Date of filing: 16.10.2023
(51) Int. Cl.: A61B 10/00, B01L 3/00

(54) **TEST DEVICE FOR DETECTING ANALYTE IN LIQUID SAMPLE**

(30) Priority: 26.09.2023 CN 202311258055
(71) Applicant: Zhejiang Orient Gene Biotech Co., Ltd, Anji Huzhou Zhejiang 313300 (CN)
(72) Inventor: LEI, Siyu, Zhejiang, 313300 (CN); FANG, Shaohua, Zhejiang, 313300 (CN); SHEN, Lili, Zhejiang, 313300 (CN); FANG, Jianqiu, Zhejiang, 313300 (CN)
(74) Representative: Piotrowicz, Pawel Jan Andrzej

(57) **Abstract**

The present invention provides a device for detecting an analyte in a liquid sample, and the device includes a sample chamber for accommodating a collector;and a detection chamber in fluid communication with the sample chamber;where the detection chamber includes a testing element therein, and the testing element is configured to detect the analyte in the liquid sample for the first time; and the sample chamberis detachably combined with the detection chamber, and the collector includes a first absorption element that is used for absorbingthe liquid sample and is capable to be compressed.

## Description

### BACKGROUND OF THE INVENTION

### Field of the Invention

The present invention relates to a device for collecting and testing a liquid sample, and in particular, to a device for collecting and testing an analyte in a liquid sample in the field of rapid diagnosis, such as a urine and saliva collection and testing device.

### Description of the Related Art

The following description is merely an introduction of some background knowledge and does not constitute any limitation to the present invention.

At present, the test device for detecting the presence or absence of an analyte in sample is widely used in hospitals or homes, and such test device for rapid diagnosis includes one or more test strips, such as early pregnancy detection and drug abuse detection. Such test device for rapid diagnosis is very convenient, and can obtain a testing result from the test strips after one minute or no at most about ten minutes. Drug detection is widely used by the drug control department, the Public Security Bureau, drug rehabilitation centers, physical examination centers, physical examination offices of national conscription, etc. The drug detection is diverse and frequent. Some detections are required to collect samples and then samples are detected in professional testing agencies or testing laboratories, and some detections need to be completed in the site in time, for example, roadsides, for example, persons who drive after drug use need to be tested on the spot (referred to as "Drug Driving"), to obtain the test results in time.

For example, the detection of saliva samples is gradually accepted and favored by testing agencies or testing personnel due to convenient collection. In some literatures, various sample collection and test devices for clinical and domestic uses have been described. For example, the US Patent No. 5,376,337 discloses a saliva sampling device in which a piece of filter paper is used for collecting saliva from the mouth of a subject and deliver saliva to an indicator reagent. US patents US 5,576,009 and US 5,352,410 each disclose a syringe-type liquid sampling device. For another example, a US patent application with the application number of 14/893,461 and publication number of US2016/0121322A1 discloses a test device for a sample; the patent only discloses some basic detection schemes and principles, and appears more difficult in the actual implementation of a specific product. For example, how to compress the pipet tip for absorbing saliva and how to move it if the cover combination is matched with the detection combination, as well as how to mix with liquid effectively, the practical effects are undesirable.

For example, collectors disclosed in US patent applications US 2020/0141934 A1, US 7,879,623 B1, US 10,564,155 B1, and US 7,879,623 B1 are connected with detection chambers, but cannot perform secondary validation; when the secondary validation needs to be performed, it is necessary to collect samples with two collectors, and then one of the collectors is used for testing, and the other thereof is used for secondary validation, so an operation is still cumbersome. In addition, such operation is not easy to implement in some cases where a sample size is small, and is cumbersome especially for saliva samples.

In view of the above technical problems in some conventional products, it is necessary to improve them and provide an alternative approach to solve the drawbacks of the prior art.

### BRIEF SUMMARY OF THE INVENTION

In view of the above situations, in order to overcome defects in the conventional technology, an objective of the present invention is to provide a device for detecting an analyte in a liquid sample, and the device can implement primary test of the liquid sample; when such detection needs to be performed and validated, a sample chamber and a detection chamber can be separated. This can realize primary sampling and two tests. In addition, an absorption element that can be compressed is used for storage and secondary sampling. The absorption element being used for storing samples during secondary test can ensure that liquid therein will not be volatilized or evaporated due to dry environment, and increase the safety of storage. For example, the liquid is not easy to leak from a container. However, a conventional container for storing secondary validation samples is an empty container, and an amount of liquid is very small; if the liquid sample is a saliva sample, the liquid sample is easy to evaporate and dry during transportation, thereby causing a sampling difficulty in the secondary validation. In the present invention, a water-absorbent element is used for absorbing and retaining liquid samples, for example, saliva samples, which can effectively preserve saliva. When the saliva is needed and tested, only the sample chamber is allowed to transport the saliva to a secondary validation center for detection, and the absorption element is allowed to be compressed to release the remaining liquid for secondary validation test.

In order to solve the problem, the present invention provides a test device, and the test device includes a sample chamber and a detection chamber, where the sample chamber is in fluid communication with the detection chamber. In some embodiments, the sample chamber is configured to receive a collector for collecting a liquid sample; and the detection chamber includes a testing element used for testing presence or absence of analytes in the liquid sample. In some embodiments, the sample chamber and the detection chamber are detachably combined, so when detection needs to be performed, the sample chamber and the detection chamber are combined and kept in fluid communication. After the detection, the sample chamber and the detection chamber are separated, such that the liquid sample in the sample chamber can be subjected to secondary assay. In some embodiments, a test strip is a lateral flow test strip based on the immune principle, and the secondary assay is a method whose sensitivity is higher than that of an immune lateral flow test, for example, liquid phase, mass spectrometry, gas phase, and other testing methods or measures having relatively high sensitivity. In some embodiments, such detachable combination means that the sample chamber and the liquid chamber are combined together at the beginning and are kept in fluid communication; after primary test, the sample chamber and the liquid chamber are separated.

In some embodiments, the detection chamber is a chamber enclosed by side walls of the detection chamber, and the chamber includes a carrier for carrying the testing element. Generally, the detection chamber is of a flat sheet structure, and the side walls of the detection chamber are provided with one or two notches; for example, a second notch or a second recess is provided in an upper end of the detection chamber; a first recess or a first notch is provided in a lower end of the detection chamber or proximal to a bottom thereof; and the notch is also similar to a tubular shape, and the first notch or the first recess is in fluid communication with the detection chamber. In some embodiments, a first protrusion and a second protrusion are externally provided in side walls of the sample chamber; the protrusions of the sample chamber can be inserted into the notches located in the side walls of the detection chamber, such that the detection chamber and the sample chamber are combined; when the detection chamber and the sample chamber are separated, the first protrusion and the second protrusion are allowed to depart from the first notch and the second notch that are located in the detection chamber to realize separation thereof. In some embodiments, the first protrusion includes a liquid channel, one end of the liquid channel is connected with the inside of the sample chamber, while the other end thereof is connected with the first notch; the first notch also includes a channel, one end of the channel is connected with the inside of the detection chamber, while the other end thereof is in fluid communication with the liquid channel of the first protrusion, such that the sample chamber is in fluid communication with the detection chamber through the liquid chamber. Thus, liquid in the sample chamber can flow into the detection chamber through the liquid channel. If the liquid channel is proximal to a bottom of the detection chamber, the liquid flows to the bottom of the detection chamber and contacts a sample application area of the testing element located at the bottom of the detection chamber, such that the liquid flows onto the testing element under capillary action to complete test or assay. Such test or assay is primary test or first test, or first assay. In some embodiments, the liquid channel is a channel without capillary force. Of course, the liquid channel can also be a channel without capillary force, and preferably the channel without capillary force. Here, the second protrusion and the second notch can be defaulted, and fitting of the second protrusion and the second notch only causes the sample chamber and the detection chamber to be more stably and firmly combined; and it is also feasible to omit the second protrusion and the second notch. In some embodiments, the detection chamber includes a sealing element for sealing an opening thereof, and the sealing element is used to allow the testing element in the detection chamber to be isolated from the outside, which prevents the testing element from test failure caused by moisture. In addition, an exhaust duct is provided on the sealing element, one end of the exhaust duct is in communication with air in the detection chamber, while the other end thereof is in communication with external atmosphere. When the liquid in the sample chamber flows into the detection chamber through the channel of the first protrusion, it generally enters the detection chamber under pressure and is not blocked by air pressure, such that the liquid can smoothly and quickly flow into the detection chamber. Pressure in the sample chamber generally increases when the absorption element of the collector is inserted into the sample chamber and the collector and an inner wall of the sample chamber are hermetically sealed to compress air in the sample chamber during the compression of the absorption element. The pressure compels liquid released from the absorption element to enter the detection chamber through the liquid channel, an exhaust duct on the detection chamber can reduce the volume of air in the detection chamber, such that the liquid can smoothly enter the detection chamber with the pressure in the sample chamber. The exhaust duct herein is usually millimeter in diameter, and can smoothly exhaust air or gas, instead of liquid.

In some embodiments, the sample chamber can further include a third protrusion, and a detachable cover is arranged on the third protrusion and used for sealing the channel of the first protrusion in the sample chamber. Thus, when the sample chamber and the detection chamber are separated, the liquid channel of the first protrusion in the sample chamber is in communication with the outside, and the first protrusion is sealed by the cover, thereby preventing the liquid in the sample chamber from leaking through the liquid channel or allowing the liquid in the sample chamber to evaporate through the liquid channel. In some embodiments, the cover and the first protrusion are meshed together through a thread structure. Thus, when the cover is combined at a partition between the sample chamber and the detection chamber, the cover cannot be found by an operator. Once the cover is separated, the liquid channel of the first protrusion in the sample chamber is not in communication with the detection chamber, but is exposed to the outside; the liquid channel is sealed by the cover, such that the remaining liquid in the sample chamber will not leak or evaporate under external dry environment.

In some embodiments, the sample chamber includes a secondary validation test chamber, and the sample chamber is used for receiving and storing the liquid sample for subsequent secondary validation test. In some embodiments, the secondary validation test chamber includes an absorption element, and the absorption element can absorb liquid and can be compressed to release the liquid. In some embodiments, a first absorption element that is located on the collector and has absorbedthe liquid sample is compressed in the sample chamber to release a part of the liquid sample, the released liquid sample is absorbed and stored by a second absorption element in the secondary validation test chamber, while an excess amount of the liquid sample flows into the detection chamber through the liquid channel. Therefore, in some embodiments, the liquid channel is in fluid communication with the secondary validation test chamber and the detection chamber. Alternatively, the liquid released by the first absorption element on the collector flows into the secondary validation test chamber, a part of the liquid flows into the detection chamber through the liquid channel for primary test, and the other part thereof is absorbed and stored by the second absorption element. When the sample chamber and the detection chamber are separated, the second absorption element is allowed to be compressed to release the liquid sample for secondary test or assay. In some embodiments, an amount of the liquid absorbed by the second absorption element is constant, that is, an amount of liquid absorbed to a saturated state is constant, while a volume of liquid absorbed by the first absorption element to a saturated state is greater than that of liquid absorbed by the second absorption element to a saturated state, where the first absorption element is located on the collector and can be compressed. Thus, an amount or a volume of liquid released by compressing the first absorption element is greater than or the volume of liquid absorbed by the second absorption element to a saturated state. This causes the excess liquid to flow into the detection chamber for the primary immunity test.

In some embodiments, after the first absorption element on the collector absorbs the liquid, the liquid released by compressing the first absorption element in the sample chamber is completely absorbed by the second absorption element, and then a first pressure is applied to the second absorption element, such that the second absorption element is compressed to release a part of the liquid; the released liquid flows into the test device through the liquid channel of the first protrusion, in this case, some chemical reagents can be treated in advance on the second absorption element, and the chemical reagents can improve the fluidity of the liquid sample; for example, the chemical reagents can destroy proteins in the liquid sample or eliminate interfering substances therein, thereby facilitating the fluidity of the liquid sample on the testing element, for example, all chemical reagents in EP2823309B 1 can be used in the present invention. After primary test and first detection, the sample chamber and the detection chamber are allowed to be separated, and then the cover on the sample chamber seals the liquid channel of the first protrusion; when secondary validation needs to be performed, the cover is removed, such that the second absorption element is subjected to a second pressure again and compressed again, thereby releasing the liquid; the released liquid also flows from the raised channel and is used for secondary test. In fact, the liquid sample absorbed by the second absorption element is compressed to release the liquid by applying the first pressure, and the released liquid is used for primary test. The other part of the liquid is compressed to release the liquid by applying the second pressure, and the released liquid is used for secondary validation test. In this case, the liquid released by the first absorption element on the collector is completely absorbed by the second absorption element; the amount of liquid absorbed by the second absorption element to a saturated state may be greater than or equal to an amount or a volume of liquid absorbed by the first absorption element to a saturated state. In some embodiments, applying pressure to the second absorption element twice is that the position of the collector in the sample chamber is changed to constantly apply pressure in stages, or may be that the collector is allowed to move as a whole to apply the first pressure to the second absorption element, and when secondary test is performed, some accessories in the collector move relative to the collector to apply the second pressure to the second absorption element. For example, after the collector is inserted into the sample chamber, the liquid sample collected by the collector is completely released to be absorbed by the second absorption element in the secondary validation test chamber, and the collector applies the first pressure to the second absorption element, such that the second absorption element is compressed to release the liquid, and the released liquid flows into the detection chamber through the liquid channel to complete primary test. After the sample chamber and the detection chamber are separated, the collector is allowed to move downward continuously to apply the second pressure to the second absorption element, such a part of the liquid is released for second detection validation. A stop block can be applied in the sample chamber, such that the collector has a first position and a second position in the sample chamber. At the first position, the collector releases the liquid and applies the first pressure to the second absorption element. At the second position, the collector applies the second pressure to the second absorption element. Generally, at the first position, the collector is distal to the bottom of the sample chamber; at the second position, the collector is proximal to the bottom of the sample chamber; in this case, the secondary validation test chamber is located near the bottom of the sample chamber and formed by sealing a part of the sample chamber through the collector, and the secondary validation test chamber includes the second absorption element.

In some embodiments, the second absorption element located in the secondary validation test chamber is higher than aninletof the liquid channel in the first protrusion; thus, when the second absorption element is compressed by the first pressure, the released liquid can flow into the liquid channel and thus into the detection chamber. In some embodiments, the second absorption element on the secondary validation test chamber has a movable cover plate with holes, and the pressure can be applied to movable cover plate to compress the second absorption element. In some embodiments, on the one hand, the cover plate is used for receiving and compressing the first absorption element to release the liquid onto the second absorption element; on the other hand, the cover plate is pressed by the first absorbent element, which compresses the second absorption element. In some embodiments, the cover plate covers the surface of the second absorption element, and when the collector is inserted into the sample chamber and the first absorption element on the collector is compressed, the released liquid flows into the second absorption element through the holes on the cover plate; in this case, the collector seals the sample chamber, such that the secondary validation test chamber and the absorption element therein are hermetically sealed, the absorption element of the collector is compressed to allow the liquid to enter the second absorption element, and the second absorption element is allowed to absorb the liquid to a saturated state. After the second absorption element absorb the liquid to a saturated state, the excess liquid on the first collector enters the second absorption element, flows into the liquid channel of the first protrusion, flows into the bottom of the detection chamber through the liquid channel, and contacts the testing element in the detection chamber, thus completing the primary test of the immune principle.

In some embodiments, the sample chamber further includes a movable chamber for receiving the collector with the first absorption element, and the first absorption element on the collector can be compressed in the movable chamber to release the liquid. In some embodiments, the secondary validation test chamber is located in the sample chamber, and the movable chamber causes the secondary validation test chamber to be in a sealing state. At the same time, the secondary validation test chamber includes an absorption element, and the second absorption element absorbs the liquid sample. In some embodiments, the secondary validation test chamber is located in the sample chamber, and hermetically sealed by the movable chamber; thus, when the movable chamber moves downward, air in the secondary validation test chamber can be compressed. This facilitates the excess liquid in the secondary validation test chamber to flow from the liquid channel of the first protrusion into the detection chamber. When the first absorption element on the collector is inserted into the movable chamber, the bottom of the movable chamber is provided with a through hole, and the absorption element is in contact with the bottom of the movable chamber to be compressed to release the liquid sample into the sealed secondary validation test chamber below the absorption element, and the second absorption element is provided in the sealed secondary validation test chamber and used for absorbing and storing a part of the liquid for secondary test. It can be understood that the collector is inserted into the movable chamber and is also hermetically sealed with the walls thereof, such that the secondary validation test chamber is sealed.

It can be understood that the so-called "sealing" in the secondary validation test chamber here does not mean that the secondary validation test chamber is completely sealed and cannot exchange any gas or liquid with the outside. The sealing here means that the entire secondary validation test chamber is sealed, and only the liquid channel is in communication with the secondary validation test chamber and the detection chamber, and it can also be considered that only the liquid channel is in fluid communication with the outside; generally, the liquid channel is used for transporting the liquid, and when the volume of the sealed secondary validation test chamber shrinks, the pressure increases, and the increased pressure compels the internal liquid to flow out through the liquid channel. Thus, an ultimate goal is to remove the liquid, and at the same time, the pressure of the sealed secondary validation test chamber is kept in balance with the outside, such that the liquid flows more smoothly, and this is a necessary condition for the implementation of the present invention.

In some embodiments, there is a space between the second absorption element and the bottom of the movable chamber, and the space is the same as the liquid channel connecting the sample chamber and the detection chamber. When the movable chamber is at the first position, the liquid released by compressing the first absorption element on the collector flows into the space, and a part of the liquid is absorbed by the second absorption element in the secondary validation test chamber or absorbed by it to a saturated state, and the other part thereof is located in the space. In this case, the movable chamber drives the collector to move downward together. This actually compresses the space of the secondary validation test chamber, compelling the liquid in the space to flow into the detection chamber through the liquid channel for primary assay or test. After the primary assay, the sample chamber and the detection chamber are separated, the cover seals an outlet of the liquid channel on the first protrusion; and when the secondary assay needs to be performed, the movable chamber is allowed to compress the second absorption element at the bottom again to release the part of the liquid. In order to enable the movable chamber to directly compress the second absorption element, a structure for applying pressure is extended at the bottom of the movable chamber and is located in the space. Thus, when the movable chamber moves downward again, the extended structure directly applies pressure to the second absorption element, such that the second absorption element is compressed to release the liquid into the space, and the space continues to be subjected to the pressure of the downward movement of the movable chamber, thereby allowing the liquid to flow into the space through the liquid channel for the secondary test of the sample.

In other some embodiments, the sample chamber and the detection chamber are an integral structure, and cannot be disassembled or separated, and the secondary validation test chamber is not located in the sample chamber, but beside the detection chamber. The secondary validation test chamber, the sample chamber, and the detection chamber are kept in fluid communication. The liquid collected by the collector is released by applying the pressure to the sample chamber, and the released liquid flows into the bottom of the detection chamber through the liquid channel connecting the detection chamber to join in the primary test of the testing element. Any excess liquid sample, if any, flows into the secondary validation test chamber, and the secondary validation test chamber includes the second absorption element, where the second absorption element absorbs the excess liquid sample for storage or preservation. When secondary validation test needs to be performed, the second absorption element can be directly removed for secondary test. In some embodiments, for ease of removal, the second absorption element is fixed onto a carrier, the carrier has a sealing element, and the sealing element is sealed with the secondary validation test chamber, such that the second absorption element is sealed in the secondary validation test chamber. When secondary validation test needs to be performed, the carrier is directly removed from the secondary validation test chamber to compress the second absorption element to release the liquid. Alternatively, after the primary test, the carrier is directly removed from the secondary validation test chamber and loaded into a small bottle; then, the small bottle is delivered or transported to a secondary validation test center. In case of test, the carrier including the second absorption element is removed from the small bottle to compress the second absorption element to release the liquid. The second absorption element including the carrier can be called a second collector. Of course, the second collector does not directly collect liquid samples (for example, the second collector absorbs saliva from the mouth of a test subject), but absorbs a part of the liquid released by compressing the first absorption element. In some embodiments, the first absorption element and the second absorption element may be both compressed and have water absorptivity, and therefore may be made of same materials or different materials; for example, the materials may be filter paper, sponge, polyester fiber and any other materials that can absorb water and be compressed to release liquid.

According to the present invention, absorption elements are used for transferring or storing secondary validation samples; thus, the present invention has the advantages that the absorption elements absorbs the liquid, which is convenient for transportation and transfer; and the loss of the liquid caused by the evaporation thereof can be avoided during transportation. A conventional secondary sampling container is used for storing a liquid sample, which may result in leakage of the liquid sample during transportation; and the liquid sample is likely to evaporate if the secondary sampling container is not sealed properly. Especially for saliva samples, they are sticky and rich in protein; if the saliva samples have a relatively small amount of liquid, such as 10-100 µL, the liquid is likely to leak or evaporate and dry in the secondary sampling container, the saliva samples cannot be sampled for the secondary test. However, if the small amount of liquid is absorbed by the second absorption element and sealed for preservation, the liquid is not easy to evaporate and dry or leak because the absorption elements have the capillary force. Therefore, the first absorption element and the second absorption element may be made of same materials or different materials. It is proved based on our experiments that 50 µL liquid stored by a container without an absorption element and 50 µL liquid absorbed by the absorption element are both placed in dry air (with humidity of 20%); in a case that a temperature is 40°C, the container stored with the liquid will dry in 24 hours due to the evaporation of the liquid, the liquid stored in the absorption element still exists and can be released about 40 µL by compressing the absorption element, and the absorption element still can be compressed to release the liquid sample after kept continuously for 120 hours, which can meet the amount used in the secondary validation. Of course, the volume of liquid absorbed by these second absorption elements for secondary validation and sample storage is quantitative, for example, any volume of samples in 10-1000 µL, which depends on an amount of liquid absorbed by the second absorption element to a saturated state. Of course, the volume of the liquid absorbed by the second absorption element to a saturated state is determined by the volume of the second absorption element (when the material is unchanged). Such volume can be freely selected, and at the same time, the second absorption element is not stored in the sealed secondary validation test chamber, so the second absorption element can be compressed during the secondary validation; alternatively, the second absorption element can be removed from the sealed secondary validation test chamber and then compressed to release the liquid. Of course, the absorption element can also be soaked in an eluent to dissolve the liquid sample or the analyte on the absorption element, and then the secondary validation can be carried out.

The present invention also includes the following specific technical solutions:

A device for detecting an analyte in a liquid sample is provided, and the device includes:
a sample chamber for accommodating a collector; and a detection chamber in fluid communication with the sample chamber; where the detection chamber includes a testing element therein, and the testing element is configured to detect the analyte in the liquid sample for the first time; and the sample chamberis detachably combined with the detection chamber.

In some embodiments, the collector includes a first absorption element that is used for absorbing the liquid sample and can be compressed.

In some embodiments, the device further includes a secondary validation test chamber, where the secondary validation test chamber includes an absorption element that can absorb the liquid sample and be compressed.

In some embodiments, the secondary validation test chamber is located in the sample chamber, and when the collector is inserted into the sample chamber, the secondary validation test chamber is hermetically sealed. In some embodiments, the first absorption element on the collector is configured to absorb the liquid sample; and when the sample chamber includes the collector, the first absorption element on the collector can be compressed to release the liquid sample, where a part of the released liquid sample can flow into the secondary validation test chamber. In some embodiments, the part of the released liquid can be absorbed and preserved by the second absorption element located in the secondary validation test chamber. In some embodiments, the liquid released by the first absorption element is completely absorbed by the second absorption element. In some embodiments, the second absorption element is allowed to release the liquid sample by applying a first pressure thereon, and the released liquid sample is used in the primary test of the testing element in the detection chamber; and the second absorption element is allowed to release a part of the liquid sample by applying a second pressure thereto, and the released liquid sample is used in the secondary validation test.

In some embodiments, the secondary validation test chamber is in fluid communication with the detection chamber. In some embodiments, a volume of liquid absorbed by the second absorption element in the secondary validation test chamber to a saturated state is constant. In some embodiments, a volume of liquid absorbed by the first absorption element is greater than a volume of liquid absorbed by the second absorption element. In some embodiments, the secondary validation test chamber is located in the sample chamber or in fluid communication with the sample chamber.

In some embodiments, the secondary validation test chamber is in fluid communication with the detection chamber through one liquid channel; alternatively, the secondary validation test chamber is in fluid communication with the sample chamber through the detection chamber and the liquid channel. Thus, the liquid flowing out of the secondary validation test chamber flows into the detection chamber through the liquid channel for primary test or secondary validation test. In some embodiments, the secondary validation test chamber is in fluid communication with the sample chamber through the detection chamber, and the liquid flowing out of the sample chamber can flow into the detection chamber for primary test, while the excess liquid flows into the secondary validation test chamber. In some embodiments, the secondary validation test chamber includes a second collector, where the second collector includes the second absorption element, and when secondary validation test needs to be performed, the second collector including the second absorption element is removed from the secondary validation test chamber, and the second absorption element is compressed to release the liquid for secondary validation.

In some embodiments, the sample chamber includes a first protrusion thatincludesthe liquid channel, and the first protrusion is detachably connected with a first recessed interface of the detection chamber, such that the sample chamber can be in detachable communication with the detection chamber through the liquid channel. In some embodiments, the sample chamber further includes a cover, and the cover is configured to seal the liquid channel when the sample chamber and the detection chamber are separated.

In some embodiments, the sample chamber includes a second protrusion, and the second protrusion is detachably connected with a second recessed interface of the detection chamber. In some embodiments, the sample chamber further includes a third protrusion, and the cover is located on the third protrusion.

In some embodiments, the sample chamber further includes a movable chamber, the movable chamber is configured to receive the collector and allow the first absorption element to be compressed to release the liquid sample, and the movable chamber has a first position and a second position in the sample chamber. In some embodiments, the collector has a bottom; the bottom and the bottom and side walls of the sample chamber are enclosed into the secondary validation test chamber, and the secondary validation test chamber includes the second absorption element for absorbing the liquid sample. In some embodiments, when the first absorption element on the collector or the first collector is compressed to release the liquid, the released liquid flows to the secondary validation test chamber, the second absorption element is allowed to absorb the liquid, and the excess liquid flows to the detection chamber through the liquid channel to contact the testing element for testing and assaying, and the movable chamber is located at the first position. In some embodiments, the collector is inserted into the movable chamber, such that pressure in the secondary validation test chamber is increased, and the increased pressure facilitates the remaining liquid to flow into the detection chamber through the liquid channel. In some embodiments, the secondary validation test chamber is in fluid communication with the detection chamber through the liquid channel. In some embodiments, after the collector and the detection chamber are separated, and when the secondary validation test needs to be performed, the movable chamber is allowed to move the second position, and the second absorption element is compressed by the movable chamber, whereby releasing the liquid through the liquid channel; and the released liquid is used for secondary validation test. In some embodiments, the detection chamber includes a carrier for carrying the testing element, the testing element is located on the carrier, and the detection chamber is in communication with external atmosphere. In some embodiments, the detection chamber includes a sealing body that seals an opening of the detection chamber, a channel is provided on the sealing body, and the channel is in communication with the detection chamber and the external atmosphere. In some embodiments, the device further includes a base, where the detection chamber and the sample chamber are located on the base and combined with the base in a detachable manner. In some embodiments, the analyte is a drug micromolecule. In some embodiments, the liquid sample is saliva, urine or blood.

Further, the present invention provides a method for testing an analyte in a liquid sample, and the method includes: performing primary test on the liquid sample, and then performing secondary validation test on the same liquid sample, where secondary validation samples are stored in the absorption element.

In some embodiments, a test device is provided, and the device includes a sample chamber for inserting a collector, and the collector includes a first absorption element that is used for absorbing a liquid sample and is inserted into the sample chamber, such that the first absorption element is compressed to release the liquid sample into the sample chamber. In some embodiments, when the collector is inserted into the sample chamber, a sealing space is formed at the bottom of the sample chamber, and the sealing space includes a second absorption element, and a part of the liquid released by the first absorption element is absorbed by the second absorption element in the sealing space, and the excess liquid is kept in the sealing space. In some embodiments, there is a liquid channel between the sealing space and the detection chamber. With the movement of the collector, pressure is applied to the sealing space, such that the liquid kept in the sealing space is compelled to flow into the detection chamber through the liquid channel for the initial detection. After the initial detection, the sample chamber and the detection chamber are allowed to be separated.

### Beneficial effect

The primary test and the secondary validation test of the analyte in the liquid chamber can be implemented through the above structure; particularly, the secondary test sample being kept in the absorption element can ensure the existence of the liquid sample to the greatest extent, rather than the reduction of the liquid sample, and the secondary validation test chamber is located in the sample chamber and can be detachably combined with the detection chamber.

### BRIEF DESCRIPTION OF THE DRAWINGS

FIG. 1 is a schematic diagram showing a three-dimensional structure of an assembled test device according to a specific embodiment of the present invention.
FIG. 2 is an exploded schematic diagram of a test device according to a specific embodiment of the present invention.
FIG. 3 is a detailed exploded schematic diagram of a test device according to a specific embodiment of the present invention.
FIG. 4 is a schematic diagram showing a cross-section structure of a test device according to a specific embodiment of the present invention (with a collector inserted into a sample chamber).
FIG. 5 is a schematic diagram showing a three-dimensional structure of a sample chamber according to a specific embodiment of the present invention.
FIG. 6 is a schematic diagram showing a three-dimensional structure of a sample chamber according to a specific embodiment of the present invention.
FIG. 7 is a schematic diagram of the movement of a sample chamber, a collector, and a second absorption element according to a specific embodiment of the present invention.
FIG. 8 is a schematic diagram showing a cross-section structure of the combination of a sample chamber and a collector according to a specific embodiment of the present invention (without a cover for sealing a liquid channel or sealing a bottom of a sample chamber).
FIG. 9 is a schematic diagram showing a three-dimensional structure of an assembled test device according to another specific embodiment of the present invention.
FIG. 10 is a schematic diagram showing an exploded structure of a test device according to a specific embodiment of the present invention.
FIG. 11 is a schematic diagram showing a three-dimensional structure of a base according to a specific embodiment of the present invention.
FIG. 12 is a schematic diagram showing an exploded three-dimensional structure of a test device according to another embodiment of the present invention.
FIG. 13 is a schematic diagram showing a cross-section structure of an assembled test device according to a specific embodiment of the present invention (test is performed when a collector is inserted into a sample chamber).
FIG. 14 is a schematic diagram showing an exploded structure of a test device according to another specific embodiment of the present invention (a sample chamber and a detection chamber include a second absorption element).
FIG. 15 is a schematic diagram showing a cross-section structure of an assembled test device according to a specific embodiment of the present invention (a sample chamber is in fluid communication with a detection chamber, a chamber including a second absorption element is in fluid communication with the detection chamber, and a secondary validation test chamber is not located in the sample chamber).
FIG. 16 is a schematic diagram showing a three-dimensional structure of a test device assembled according to another embodiment of the present invention.
FIG. 17 is a schematic diagram showing an exploded structure of a test device according to a specific embodiment of the present invention.
FIG. 18 is a schematic diagram showing an exploded structure of a sample chamber, a collector, and a movable chamber according to a specific embodiment of the present invention.
FIG. 19 is a schematic diagram showing a cross-section structure of a collector being located in a movable chamber and the movable chamber being located in a sample chamber according to a specific embodiment of the present invention.
FIG. 20 is a top view of a testing element.
FIG. 21 is a schematic diagram showing a three-dimensional structure of a testing element.

### DETAILED DESCRIPTION OF THE INVENTION

The following further describes the structures involved in the present invention or the technical terms used therein. Unless otherwise specified, they shall be understood and explained according to the general terms commonly used in the prior art.

### Detection

Detection means assaying or testing presence or absence of a substance or material, including but not limited to, chemical substance, organic compound, inorganic compound, metabolite, drug, drug metabolite, organic tissue, metabolite of organic tissue, nucleic acid, protein or polymer. In addition, detection means that the amount of a substance or material is tested. Further, assay also means immunoassay, chemical assay, enzyme assay, and the like.

### Sample

The samples detected by the test device of the present invention include biological liquid (for example, case liquid or clinical sample). Liquid samples or fluid samples may be derived from solid or semi-solid samples, including feces, biological tissues and food samples. The solid or semi-solid samples may be converted to liquid samples by any appropriate methods, such as mixing, mashing, macerating, incubating, dissolving, or digesting the solid samples by enzymolysis in suitable solutions, such as water, phosphate solutions, or other buffer solutions. "Biological samples" include animal, plant, and food derived samples, including, for example, human or animal derived urine, saliva, blood and components thereof, spinal fluid, vaginal secretions, sperm, feces, sweat, secretions, tissues, organs, tumors, cultures of tissues and organs, cell cultures, and media. Preferably, the biological sample is urine, and preferably, the biological sample is saliva. Food samples include food processed materials, final products, meat, cheese, wine, milk, and drinking water. Plant samples include samples derived from any plants, plant tissues, plant cell cultures, and media. "Environmental samples" include samples derived from the environment (e.g., liquid samples from lakes or other bodies of water, sewage samples, earthen samples, groundwater, seawater, and waste liquid samples). The environmental sample may further include sewage or other waste water.

An appropriate test device according to the present invention can be used to detect any analyte. Preferably, the test device of the present invention is used to detect small drug molecules in saliva and urine. Of course, the samples detected by the test device of the present invention may be any samples of the above forms, regardless of being solid or liquid at the beginning, provided that these liquids or liquid samples can be absorbed by the sample application area of the testing element. Generally, the sample application area is made of a water absorbent material, and liquid samples or fluid samples can be absorbed by the capillary or other characteristics of the material of an absorption element, so that the liquid sample can flow in the sample application area. The material of the sample application area may be any material capable of absorbing liquid, such as sponge, filter paper, polyester fiber, gel, non-woven fabric, cotton, polyester film, and yarn. Of course, the sample application area may be made of a water absorbent material or a non-water absorbent material. However, the absorption element is provided with holes, screw threads, and caves on which the samples can be collected. Generally, the samples are solid or semi-solid samples, and filled between screw threads and in the holes or caves for collection. Of course, optionally, the sample application area may be composed of some non-absorbent fibers and hairs, and these materials are used to scrape a solid, semi-solid or liquid sample, so that these samples can be retained on the sample application area. If detection needs to be performed, a buffer solution is applied to the sample application area to dissolve the sample, so that the dissolved sample flows on the testing element or the detection element.

In some embodiments, the liquid sample is located in the detection chamber, instead of being manually applied to the sample application area of the testing element of the present invention, where the sample application area is proximal to the bottom of the detection chamber; when there is the liquid sample at the bottom of the detection chamber, an end portion of the sample application area contacts the liquid sample, the liquid sample sequentially flows into the label area, the testing area, and the sample absorption area depending on the capillary force, thereby completing whole detection or primary test or first test.

### Downstream and upstream

Downstream or upstream is divided according to a flow direction of a liquid, generally, a liquid or fluid flows to a downstream area from an upstream area. The downstream area receives the liquid from the upstream area, and a liquid also may flow to a downstream area along an upstream area. Here, downstream or upstream is generally divided according to a flow direction of a liquid, for example, on some materials where capillary force is utilized to facilitate the flow of a liquid, a liquid may overcome gravity to flow towards an opposite direction to the gravity; and in this case, downstream or upstream is divided according to a flow direction of the liquid. For example, in the test device of the present invention, when there is the liquid sample at the bottom of the detection chamber, for example, the liquid sample from the sample chamber, such as a urine sample or a saliva sample of a test subject, can flow from the sample application area to the label area, and then to the testing area, for example, a test result area and a test result control area. The testing area may be a polyester fiber film, and the diversion element may be a glass fiber, a polyester chip, and a polyester film.

### Gas flow or liquid flow

Gas flow or liquid flow means that liquid or gas can flow from one place to another place. In a flow process, the liquid or gas may pass through some physical structures to play a guiding role. The "passing through some physical structures" here means that liquid passes through the surface of these physical structures or their internal space and flows to another place passively or actively, where passivity is usually caused by external forces, such as flow under the capillary action and the action of air pressure. The flow here may also be a flow due to self-action (gravity or pressure) of the liquid or gas, and also may be a passive flow. The fluid under the action of air pressure may be a forward flow, or also a reverse flow; or a fluid is urged to flow to another position from a position under the action of air pressure. Here, the flow does not mean that a liquid or a gas is necessarily present, but indicates a relationship or state between two objects under some circumstances. In case of presence of liquid, it can flow from one object to another. Here it means the state in which two objects are connected. In contrast, if there is no gas flow or liquid flow state between two objects, and liquid exists in or above one object but is unable to flow into or on another object, it is a non-flow, non-liquid or non-gas flow state.

### Testing element

The "testing element" used herein refers to an element that can be used to detect whether a fluid sample or a fluid specimen (a liquid sample or a liquid specimen) contains an interested analyte. Such testing can be based on any technical principles, such as immunology, chemistry, electricity, optics, molecular science, nucleic acids, and physics. The testing element can be a lateral flow detection reagent strip that can detect a variety of analytes. Of course, other suitable testing elements can also be used in the present invention.

Various testing elements can be combined for use in the present invention. One form of the testing elements is a test strip. The test strips used for analyzing the analyte (such as drugs or metabolites that show physical conditions) in samples can be of various forms such as immunoassay or chemical analysis. The analysis mode of non-competition law or competition law can be applied for test strips. A test strip generally contains a water absorbent material that has a sample application area, a reagent area, and a testing area. Fluid or liquid samples are added to the sample application area and flow to the reagent area under the capillary action. If analyte exists in the reagent area, samples will bind to the reagent. Then, samples continue to flow to the testing area. Other reagents such as molecules that specifically bind to analyte are immobilized on the testing area. These reagents react with the analyte (if any) in the sample and bind to the analyte in this area, or bind to a reagent in the reagent area. Label used to display the detection signal exists in the reagent area or the detached label area.

Typical non-competition law analysis mode: if a sample contains analyte, a signal will be generated; and if not, no signal will be generated. Competition law: if no analyte exists in the sample, a signal will be generated; and if analyte exists, no signal will be generated.

The testing element can be a test strip, which can be water absorbent material or non-water absorbent material. The test strip can contain several materials used for delivery of liquid samples. One material of the test strip can cover the other material thereof. For example, the filter paper covers the nitrocellulose membrane. One or more materials may be used in one area of the test strip, and one or more other different materials may be used in the other area thereof. The test strip can stick to a certain support or on a hard surface for improving the strength of holding the test strip.

Analyte is detected through a signal generating system. For example, one or more enzymes that specifically react with this analyte is or are used, and the above method of fixing a specific binding substance on the test strip is used to fix the combination of one or more signal generating systems in the analyte testing area of the test strip. The substance that generates a signal can be in the sample application area, the reagent area or the testing area, or on the entire test strip, and one or more materials of the test strip can be filled with this substance. The solution containing a signifier is added onto the surface of the test strip, or one or more materials of the test strip is or are immersed in a signifier-containing solution. The test strip containing the signifier solution is made dry.

Various areas of the test paper can be arranged as follows: sample application area 405, reagent area 407, and testing area 408, where the testing area includes a test result area 411 and a test result control area 410. The test result control area is located behind the testing area. All areas can be arranged on a test strip that is only made of one material. Alternatively, different areas may be made of different materials. Each area can be in direct contact with the liquid sample, or different areas are arranged according to the flow direction of liquid sample; and a tail end of each area is connected and in overlapped with the front end of the other area. Materials used can be those with good water absorption such as filter papers, glass fibers or nitrocellulose membranes. The test strip can also be in other forms.

The nitrocellulose membrane test strip is commonly used, that is, the testing area includes a nitrocellulose membrane (NC) on which a specific binding molecule is immobilized to display the test result; and other test strips such as cellulose acetate membrane or nylon membrane test strips can also be used. For example, test strips and similar devices with test strips disclosed in the following patents: US 4857453; US 5073484; US 5119831; US 5185127; US 5275785; US 5416000; US 5504013; US 5602040; US 5622871; US 5654162; US 5656503; US 5686315; US 5766961; US 5770460; US 5916815; US 5976895; US 6248598; US 6140136; US 6187269; US 6187598; US 6228660; US 6235241; US 6306642; US 6352862; US 6372515; US 6379620, and US 6403383. The test strips and similar device with test strips disclosed in the above patents may be applied to the testing element or test device of the present invention for the detection of an analyte, for example, the detection of an analyte in a sample.

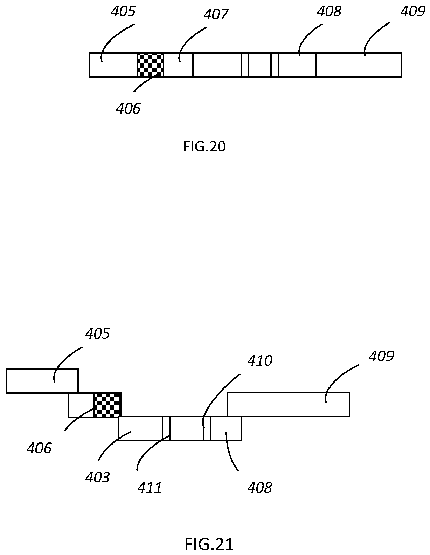

Test strips used in the present invention may be commonly referred as lateral flow test strips. The specific structure and detection principle of the test strips are well known to a person skilled in the art in the prior art. A common test strip (FIG. 20-FIG. 21) includes a sample collection area or a sample application area 405, a label area (406), and a testing area 408. The sample collection area includes a sample receiving pad, the label area includes a label pad, and the water absorption area may include a water absorption pad, where the testing area includes necessary chemical substances that can detect presence or absence of the analyte, such as an immune reagent or an enzyme chemical reagent. As shown in FIG. 21, the test strip has the sample application area 405. The nitrocellulose membrane test strip is commonly used, that is, the testing area 408 includes a nitrocellulose membrane, and an area 411 (T-line) on which a specific binding molecule is immobilized to display the test result; and other test strips such as cellulose acetate membrane or nylon membrane test strips can also be used. Of course, in the downstream of the testing area, there may also be a test result control area 410 (C-line); generally, test strips appear on the test result control area and the testing area in the form of a horizontal line, namely, a test line or a control line. Such test strips are conventional. Of course, they can also be other types of test strips for detection under the capillary action. In addition, there are dry chemical reagent components on common test strips, for example, an immobilized antibody or other reagents. When the test strip contacts liquid, the liquid flows along the test strip under the capillary action, and the dry reagent components are dissolved in the liquid and treated in a next area, and the dry reagents react in the area for necessary detection. The liquid flow mainly relies on the capillary action. Here, all of the test strips can be applied to the test device of the present invention or can be disposed in contact with the liquid samples in a detection chamber or used to detect the presence or absence of analyte in the liquid samples that enter a detection chamber, or the quantity thereof.

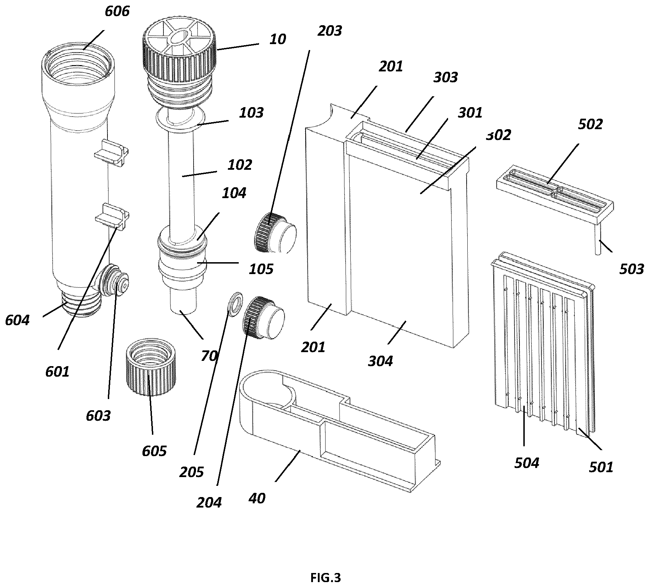

In addition to the foregoing test strip or lateral flow test strip which is used to contact with the liquid sample to test whether the liquid samples contain analytes. The testing element of the present invention may be used as a test device by itself to detect an analyte in a sample. Therefore, the test device here is equal to a testing element. For example, after being mixed with a treatment solution, the fluid sample is detected with a testing element directly, specifically described as follows: when a receiving device is described to treat a fluid sample, the testing element may be used for detection alone. In the present invention, the testing element is arranged on a carrier 501, and the carrier is provided with a plurality of grooves 504, and each of the grooves is provided with a testing element that can be used for testing an analyte, as shown in FIG. 3, and the carrier is provided with a plurality of grooves that can be used for detecting or testing a plurality of analytes. Generally, the sample application area 405 is located at a lower end of the groove proximal to the carrier, and when the carrier is inserted into the detection chamber, an end portion of the sample application area is generally an area that is in contact with a bottom 304 of the detection chamber; thus, when flowing into the detection chamber, test liquid flows into the bottom thereof, and the sample application area of the detection chamber contacts the liquid sample to complete the primary test.

### Analyte

Examples that can use an analyte related to the present invention include some smallmolecule substances, including drugs (such as drug of abuse). "Drug of Abuse" (DOA) refers to using a drug (playing a role of paralyzing the nerves usually) not directed to a medical purpose. Abuse of these drugs will lead to physical and mental damage, dependency, addiction and/or death. Examples of drug abuse include cocaine; amphetamine (AMP) (e.g., Black Beauty, white amphetamine tablets, dexamphetamine, dexamphetamine tablets, and Beans); methamphetamine (MET) (crank, meth, crystal and speed); barbiturate (BAR) (such as Valium, Roche Pharmaceuticals, Nutley, and New Jersey); sedatives (i.e., a sleep aid medicine); lysergic acid diethylamine (LSD); inhibitors (downers, goofballs, barbs, blue devils, yellow jackets, and methaqualone); tricyclic antidepressants (TCAs, i.e. imipramine, amitriptyline, and doxepin); dimethylenedioxymethylaniline (MDMA); phencyclidine (PCP); tetrahydrocannabinol (THC, pot, dope, hash, weed, etc.); opiates (i.e., morphine (MOP) or opium, cocaine (COC), heroin, and hydroxydihydrocodeinone); and anxiolytic drugs and sedative-hypnotic drugs. The anxiolytic drugs are mainly used for relieving anxiety, tension, and fear, and stabilizing emotion, and have hypnotic and sedative effects. The anxiolytic drugs include benzodiazepines (BZO), atypical benzodiazepines (BZ), fused dinitrogen NB23C, benzodiazepines, ligands of BZ receptors, openring BZ, diphenylmethane derivatives, piperazine carboxylates, piperidine carboxylates, quinazolinones, thiazine and thiazole derivatives, other heterocycles, imidazole-type sedative/analgesic drugs (e.g., oxycodone (OXY) and methadone (MTD)), propylene glycol derivatives-carbamates, aliphatic compounds, anthracene derivatives, and the like. The test device of the present invention may also be used for detecting drugs belonging to a medical use but easy to be taken excessively, such as tricyclic antidepressants (imipramine or analogues) and acetaminophen. These drugs are metabolized into micromolecular substances after absorbed by human body. These micromolecular substances exist in blood, urine, saliva, sweat and other body fluids or in some body fluids.

For example, the analyte detected by the pre invention includes but is not limited to creatinine, bilirubin, nitrite, (nonspecific) proteins, hormones (for example, human chorionic gonadotropin, progesterone, follicle-stimulating hormone, etc.), blood, leucocytes, sugar, heavy metals or toxins, bacterial substances (such as proteins or carbohydrates against specific bacteria, for example, *Escherichia coli* 0157:H7, *Staphylococcus, Salmonella, Fusiformis, Camyplobacter genus, L. monocytogenes, Vibrio,* or *Bacillus cereus*) and substances related with physiological features in a urine sample, such as pH and specific gravity. Chemical analysis of any other clinical urine may be performed by lateral flow test in combination with the device of the present invention. The sample of the present invention may be urine, and the analyte may be HCG, LH, and other substances, which are used for testing ovulation or early pregnancy.

### Detachable combination

Detachable combination means that connection relationship between two components is in several different states or positional relationship. For example, with two components being physical components, they can be separated at the beginning and then connected or combined in an appropriate first case, and separated in an appropriate second case. Physically, such separation is spatial separation without contact. Alternatively, the components are combined at the beginning, and can be physically separated from each other when appropriate. Alternatively, two objects are separated at the beginning, combined to achieve a specified function if necessary, then separated, or later combined again for a purpose. In short, combination or separation or two components or two objects can be easily made and repeated many times. Of course, the combination or separation can also be single-use. In addition, such combination can be a detachable combination between two components, or a two-by-two detachable combination between three or more components. For example, a first component, a second component, and a third component are provided, where a detachable combination is made between the first component and the second component or between the second component and the third component; and a detachable combination or separation is made between the first component and the third component. In addition, for the combination, two objects themselves can be detached or can be indirectly combined by other objects.

### Sample preservation after secondary validation test

Second validation here means that after primary test on a sample is performed, it is hoped that secondary test on the sample is performed for validation. The samples for the primary test are the same as those for the secondary validation test. Here, the same samples refer to a same batch of samples, for example, a part of obtained samples are used for the primary test and the other part thereof are used for the secondary validation test. In some embodiments, the accuracy or sensitivity of primary test is lower than that of the secondary validation test. For example, the primary test is performed using an immune-based method for a lateral flow testing element according to the present invention, and a label is a colored particle. If the secondary validation test needs to be performed, a method with better accuracy than immunoassay is used; for example, chromatography, fluorescence immunoassay, radioimmunoassay, gas phase or liquid phase, or gas/liquid phase-mass spectrometry is used for detection. It can be understood that the primary test and the secondary test share methods, for example, the primary test is an immune test, and the secondary test can also be the same as the immune test of the primary test.

### Sample chamber, detection chamber, and secondary validation test chamber

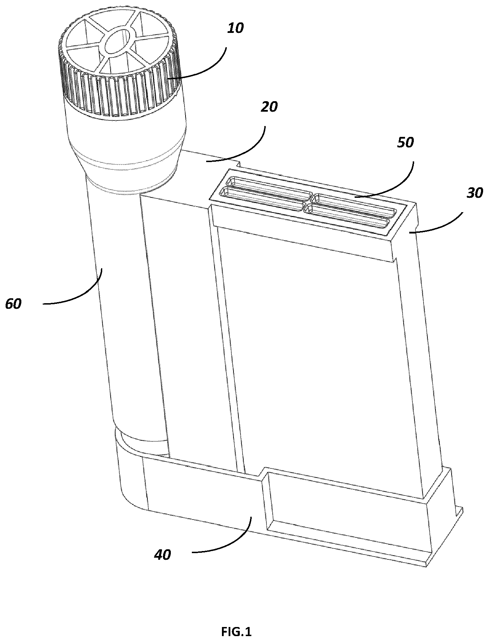

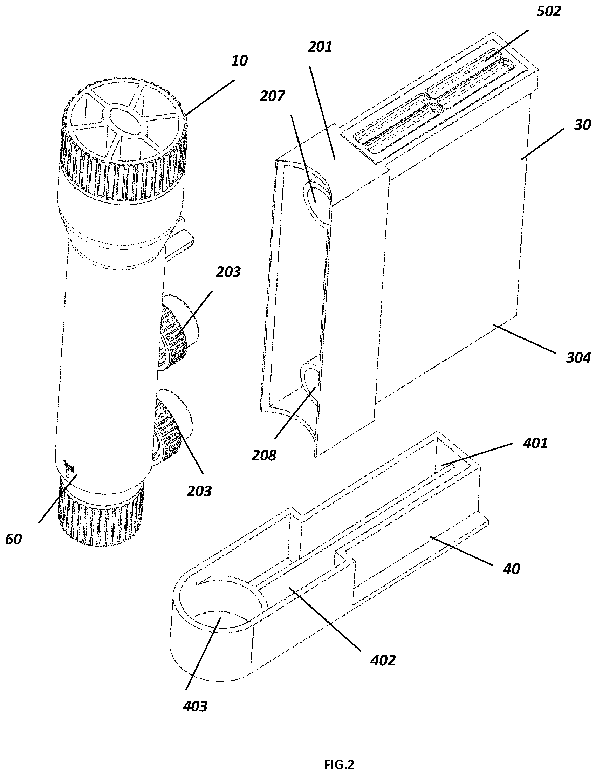

A specific embodiment of the present invention is as shown in FIG. 1-FIG. 8. The test device of the present invention includes a sample chamber 60 and a detection chamber 30, where the detection chamber 30 is detachably combined with the sample chamber 60. Generally, at the beginning, the sample chamber and the detection chamber are combined together; and after the primary test, the sample chamber and the detection chamber can be separated. In some embodiments, the sample chamber and the detection chamber that are combined are located together on a base 40, and the base is detachably combined with the sample chamber and the detection chamber that are combined. In other words, the sample chamber, the detection chamber, and the base are all detachably combined. As shown in FIG. 2 and FIG. 3, the sample chamber 60 includes a chamber with an opening 606 for inserting a collector and collecting a liquid sample on the collector. The chamber is provided with two insertion structures 601, 603 that extend outward from walls of the sample chamber 60 and are similar to protrusions; and two corresponding recesses 207, 208 are provided near the detection chamber 30 and mainly used for inserting protrusions 601, 603 of the sample chamber, thereby allowing the sample chamber and the detection chamber to be connected together. In some embodiments, the recess 208 is located near a bottom of the detection chamber and is in fluid communication with the detection chamber; a liquid channel 607 is provided in the protrusion 603 of the sample chamber; one end of the liquid channel is in fluid communication with the sample chamber, and the other end thereof is in communication with the liquid channel in the recess 208 or is directly in fluid communication with the detection chamber; thus, the liquid in the sample chamber can flow into the detection chamber 30 through the liquid channel 607 and contact the testing element in the detection chamber to complete primary test. When the sample chamber is in communication with the detection chamber 30, it is undesired that a specific communication mode is seen from the outside; a transitional protection area 20 is arranged between the sample chamber and the detection chamber and may be directly connected to the detection chamber 30 or the sample chamber 60, which completely hides a connection structure of the sample chamber and the detection chamber. In addition, the sample chamber further includes a protrusion 602, where the protrusion is not corresponding to the recess on the detection chamber, but is used for accommodating a cover 203. When the sample chamber and the detection chamber are connected together, the cover is hung on the protrusion 602 and is also hidden by the transitional protection area 20. It can be understood that the protrusion 601 in the sample chamber 60 and the recess 207 in the detection chamber 30 can be defaulted, and the protrusion 603 with a channel in the sample chamber can fit with the recess 208 in the detection chamber, or the sample chamber is detachably combined with the detection chamber. When the sample chamber and the detection chamber are separated, the protrusion 603 in the sample chamber is detached from the recess 208 in the detection chamber to realize separation thereof. After the sample chamber and the detection chamber are separated, and because the liquid channel 607 in the protrusion 602 is exposed, the cover located in the protrusion 603 can be disassembled and sleeved on the protrusion 603 to seal the liquid channel 607. This prevents the liquid in the sample chamber from leakage and polluting the environment because the liquid flows into the outside; and the liquid flowing into the outside cause pollutions to test operators. Of course, in order to insert the protrusion 603 into the recess 208 in the detection chamber to play a better connection role, an elastic sealing ring 205 is provided on the protrusion; contact with the elastic sealing ring can allow the protrusion 603 and the recess to form liquid sealing, the liquid channel is connected with the detection chamber, and the liquid in the sample chamber can safely flow into the detection chamber. In some embodiments, the detection chamber has a chamber including a carrier element 501, and a plurality of grooves are formed on the carrier and used to accommodate the testing element. The detection chamber has a front face 302 and a back face 303 that are of a planar structure and enclose into a chamber. The carrier is inserted into the chamber through the opening 301 of the chamber, and then a sealing cover 502 is arranged on the detection chamber and used to seal the opening 301 of the detection chamber and limit the position of the carrier. Optionally, the carrier includes a channel 503, and the channel 503 extends from the sealing cover to the inside of the detection chamber, such that gas in the detection chamber can be kept in communication with the external atmosphere through the channel 503, which helps the liquid in the sample chamber to enter the detection chamber through the channel 607 without additional pressure obstruction. The detection chamber has a bottom 304, where the bottom is located in the groove 401 of the base 40 to maintain the stability of the detection chamber, and the base may be defaulted and is to make the whole test device stand during the test. However, the transitional protection area 20 also has a top 201 and a bottom 202, where the bottom is located in a groove area 402 of the base 40, and a part of the sample chamber is located in a recessed area 403 of the base 40, such that the sample chamber, the detection chamber, and the transition area connecting the detection chamber and the sample chamber are located on the base. The base is detachably combined with the sample chamber 60 and the detection chamber 30, and the base can make the sample chamber and the detection chamber be vertical. Actually, the cover bodies 203, 204 in FIG. 1-FIG. 8 are of a same cover, but they are only located at different positions for distinction. At the beginning, the cover is located on the protrusion 602. After the sample chamber 60 and the detection chamber 30 are separated, the cover 203 seals the protrusion 603 to seal the channel 607.

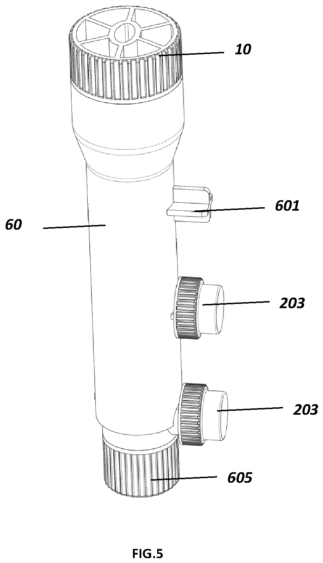

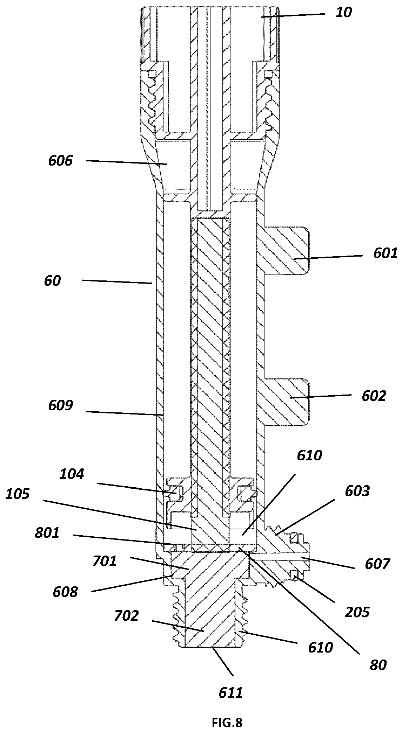

The structure of the sample chamber and the structure of the collector are as shown in FIG. 5-FIG. 8. In some embodiments, the sample chamber 60 is used to receive the collector, allow the collector to be inserted into the sample chamber, and release the liquid into the sample chamber. The sample chamber has openings 606, 611 at two ends thereof, where the opening 606 at one end thereof is used to insert the collector and is sealed by cooperating with a hand-held portion 101 of the collector, and the opening 611 at the other end thereof is sealed by a cover 605. The cover 605 is meshed with threads on outer walls 610 of the opening 611 at one end of the sample chamber in the form of threads. In addition, the chamber further includes a baffle 80, where the baffle is located in the sample chamber and proximal to the bottom of the sample chamber; thus a secondary validation test chamber 608 is formed between the baffle 80 and the opening 611; the chamber includes an absorption element 70, and the absorption element is used to absorb the liquid sample in the sample chamber, and the secondary validation test chamber 608 is kept in fluid communication with the detection chamber through the liquid channel 607. An area between the baffle 80 and the opening 606 of the sample chamber is used to insert the collector. The collector 10 includes the hand-held portion 101 and a connecting rod connected with the hand-held portion 101, where an elastic sealing ring 104 is arranged on one end of the connecting rod, and a first absorption element 105 is arranged at a lower part of the elastic sealing ring, used to absorb the liquid sample, and can be compressed; when the collector is inserted into the sample chamber, the baffle 80 is in contact with the first absorption element of the collector; with the collector being inserted into the sample chamber, the first absorption element is compressed to release the liquid, the baffle is provided with an opening 801 through which the liquid flows into the chamber 608 below the baffle. In some embodiments, the chamber 608 between the baffle and the opening 611 is completely occupied by the second absorption element (as shown in FIG. 8), the baffle does not move, and at the same time, the chamber 608 is also in communication with the channel 607 on the protrusion 603; alternatively, the opening at one end of the liquid channel 607 is located between the baffle 80 and the opening 611 at one end of the sample chamber to realize liquid communication with the secondary validation test chamber 608. The liquid released by compressing the first absorption element 105 on the collector when the first absorption element contacts the baffle is completely absorbed by the second absorption element 70. When the second absorption element absorbs the liquid to a saturated state, the excess liquid passes through the liquid channel 607 through the second absorption element and flows into the bottom of the detection chamber, and can be discharged only through one outlet.

This represents the discharge capacity of the liquid channel 607, and the excess liquid can flow out of the sample chamber. In order to allow the excess liquid to flow into the detection chamber smoothly through the second absorption element and the liquid channel 607, the elastic sealing element 104 on the collector and an inner wall 609 of the sample chamber are hermetically sealed, which allows the secondary validation test chamber to be sealed; when the first absorption element is compressed, the elastic sealing element 104 also moves, thereby forming a pressure (when the volume of the sealing space 608 decreases continuously, the pressure increases, and the baffle is located in the sealing space) applied to a sealing space between the elastic sealing element 104 and the opening 611 of the sample chamber (in this case, the opening is sealed by the cover 605). This pressure facilitates the liquid released by compressing the first absorption element to flow into the second absorption element. After the second absorption element absorbs the liquid to a saturated state, the excess liquid is also subjected to the pressure and flows into the detection chamber through the liquid channel 607, and the detection chamber has the channel 503 in pressure communication with the outside, such that a pressure difference exists between the sealing chamber 608 and the detection chamber, and the pressure difference causes the excess liquid to flow into the detection chamber through the second absorption element and the liquid channel 607. Here, in fact, the secondary validation test chamber 608 has two functions, where one function is to collect the liquid released by compressing the absorption element on the collector, and the other function is to give play to intermediary transmission. In the secondary validation test chamber 608, a part of the liquid is absorbed by the second absorption element to a saturated state, and the rest of the liquid (which cannot be absorbed by the second absorption element) normally flows to the detection chamber through the liquid channel 607 to contact the testing element therein to complete the primary test. Here, a volume of liquid absorbed by the second absorption element to a saturated state is less than a volume of the liquid released by compressing the absorption element on the collector, such that the excess liquid flows into the detection chamber.

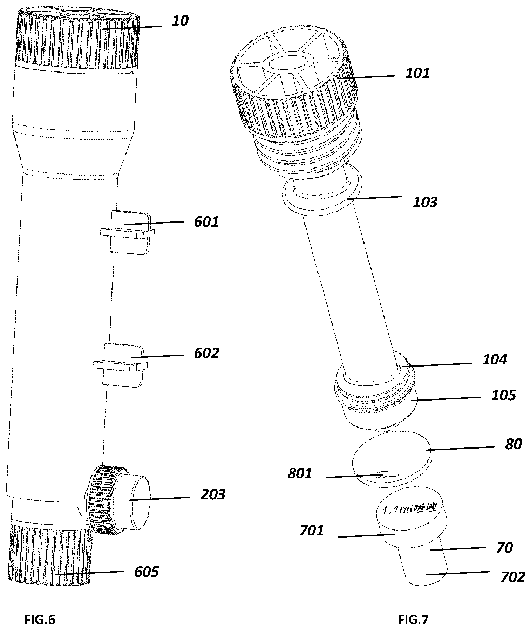

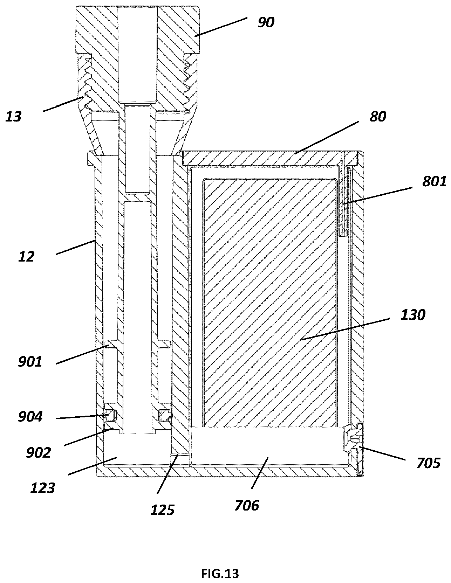

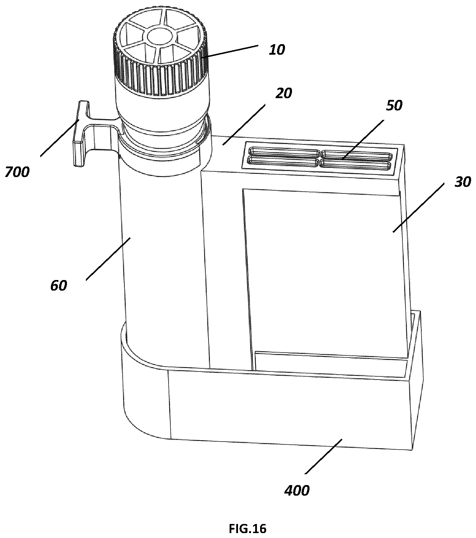

After the test, the sample chamber and the detection chamber are allowed to be separated, such that the cover 203 located on the protrusion 602 is meshed with the outside of the protrusion 603, thereby sealing the liquid channel 607; then, the entire sample chamber 60 is allowed to be separated from the base 40, as shown in FIG. 6. In this case, the collector 10 is located in the sample chamber 60 and the second absorption element is also located in the sample chamber. In this case, if the sample needs to be subjected to secondary validation test, the entire sample chamber can be delivered to a professional laboratory for secondary test. During the test, there are two ways. One way is to allow the cover 605 to be detached from the sample chamber, thus exposing the second absorption element 70. Because the second absorption element is both absorbable and compressible, the second absorption element 70 can be compressed, thus releasing the liquid for the second validation test. There are several ways to allow the second absorption element to be compressed. One way is to continue to rotate the collector, such that the collector continues to move downward in the sample chamber. In this case, the baffle 80 also moves. The movement of the baffle compels the second absorption element to be compressed, such that the second absorption element releases the liquid due to compression. The released liquid can flow out of the liquid channel 607 (in this case, the cover 203 needs to be removed, but the cover 605 is not removed). The liquid flowing out of the liquid channel 607 can be used for secondary validation, and the continuous compression of the collector can be controlled, that is, the depth of the collector inserted into the sample chamber is controlled, a first insertion depth thereof is to complete the primary test; if a further insertion depth is longer, the second absorption element is compressed to release the liquid for secondary validation. Here, how the controller is controlled can be explained with reference to FIG. 13. The sample chamber also includes a control structure 13 located at an inlet 121 of the sample chamber, and the control structure is similar to a small chamber 13 extending from the inlet of the sample chamber, such that the collector is inserted into the opening 132 of the control structure and enters into the sample chamber to compress the absorption element thereon; when the secondary test needs to be performed, the controller 13 is removed from the inlet of the sample chamber, the collector can continue to move downward more proximal to the bottom of the sample chamber and can apply the pressure to the second absorption element to release the liquid for the secondary test. The controller can also be a snap ring 700 as shown in FIG. 16, which can realize the above objectives of the present invention.

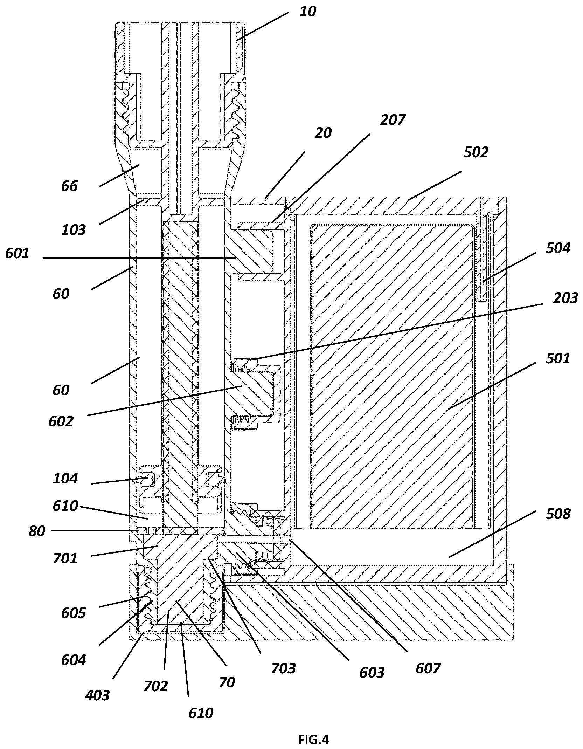

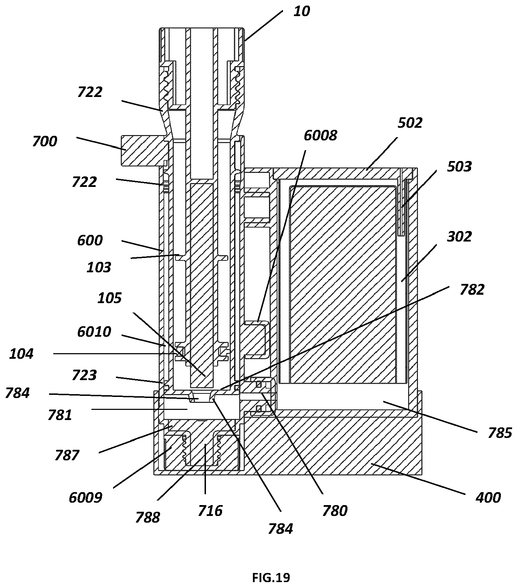

The other way is to remove the cover 605 instead of the cover 203. The absorption element has a step 680 in the sealed secondary validation test chamber 608 in the sample chamber, where the step has a big end 701 and a small end 702. The secondary validation test chamber 608 is fully filled with the second absorption element, and the big end 701 cannot be compressed again when compressed to the surface of the step, so the volume of the entire absorption element is reduced, and the second absorption element 70 absorbs the liquid to a saturated state. When the big end 701 is compressed, the liquid released by it normally flows out of the opening 611 of the sample chamber through the small end 702 and may be used for secondary validation test. In the above ways, generally, the volume of liquid absorbed by the second absorption element to a saturated state is constant, that is, the second absorption element can be up to a saturated state only by absorbing a specified volume of liquid instead of more liquid. However, the volume of the liquid absorbed by the first absorption element or the volume of the liquid released by compressing the first absorption element is greater than the volume of the liquid absorbed by the second absorption element to a saturated state, such that the excess liquid can flow into the detection chamber for the primary test. In some embodiments, a space under the baffle is not completely occupied by the second absorption element, but there is a space 785(with reference to FIG. 19) between the second absorption element and the baffle, and the space is also in fluid communication with the liquid channel 607; in this case, the baffle is not movable. When the first absorption element of the collector is inserted into the sample chamber, the first absorption element contacts the baffle and is compressed to release the liquid sample. In this case, the liquid flows into the space and then is allowed to be absorbed by the second absorption element to a saturated state, and the excess liquid directly flows into the detection chamber through the liquid channel 607, such that the liquid can flow into the detection chamber smoothly without being blocked by the second absorption element. After the sample chamber is detached from the detection chamber 30 and the base 40, in order to complete the secondary validation test, the cover 605 can be removed, one tool is used to reversely apply the pressure to the second absorption element, for example, one tool is used to apply the pressure to the opening 611 to compress the second absorption element to release the liquid, and the released liquid flows out of the liquid channel 607 for the secondary test. Of course, in order to allow the collector and the sample chamber to form good seal fitting, the collector further can include a second sealing element 103, where the second sealing element 103 and the inner wall of the sample chamber can also be hermetically sealed; thus, when the collector is inserted into the sample chamber and the absorption element contacts a shift lever, the absorption element can be better compressed and the liquid released by the absorption element flows into the secondary validation test chamber below the absorption element as much as possible (FIG. 4).

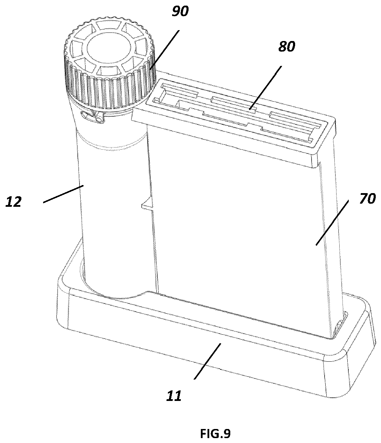

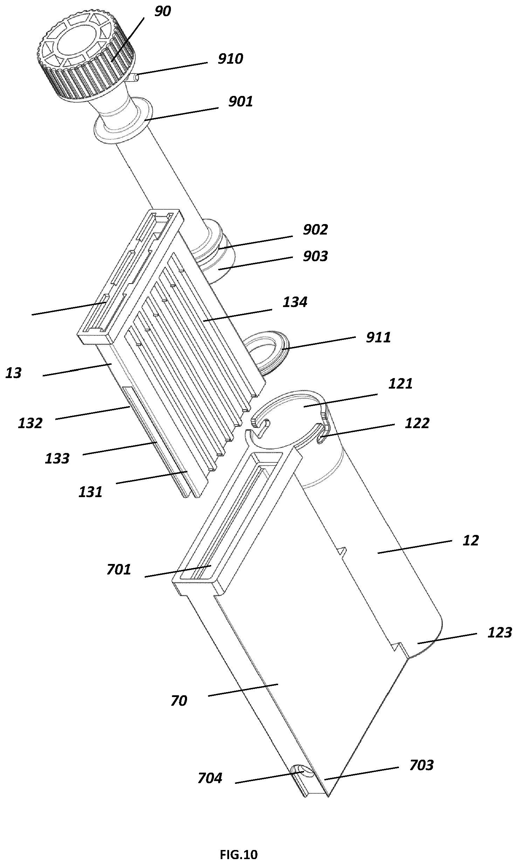

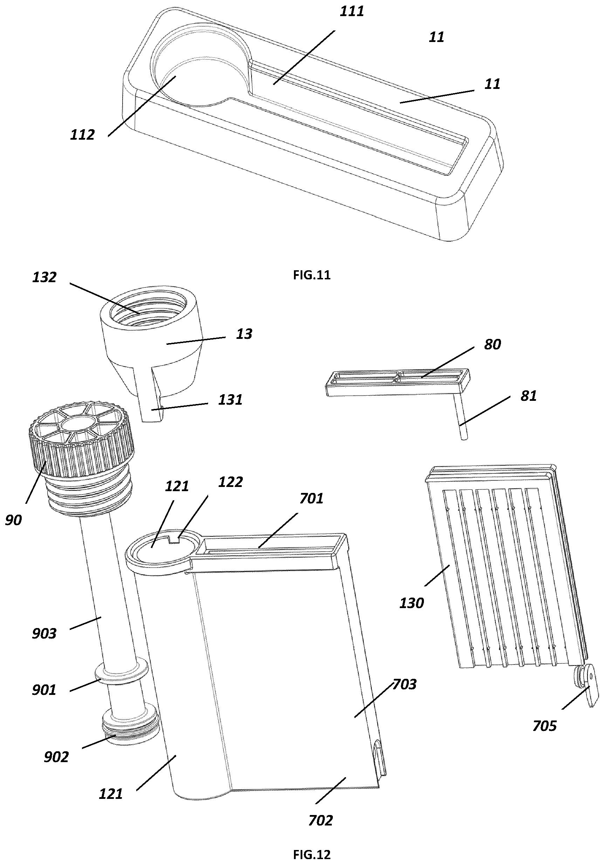

FIG. 9-FIG. 13 show another specific embodiment. The test device includes a sample chamber 12, a collector 90, a detection chamber 70, and a base 11, which are combined together into an integral test device or system. At the beginning, the collector is packaged independently, and the sample chamber includes an opening 121 with a locking structure, such as a positioning rotation lock; and the collector has an insertion element 910; when the insertion element is aligned with an opening of a keyhole 122, the depth of the collector inserted into the sample detection is fixed by rotation. The collector has two sealing elements, namely a sealing element 901 proximal to the cover and a sealing element 911 proximal to the first absorption element. The sealing elements are clamped on a groove 902, and a first absorption element 903 is proximal to the bottom of the groove. In the embodiment, the carrier is composed of panels; the panels have a thickness and are provided with one groove 133, such that the panels are divided into a front portion 131 and a rear portion 132; a plurality of grooves are formed in the front of each panel and used to load the testing element; and the sample application area of the testing element protrudes from the grooves, and ends of the grooves are in contact with the bottoms 702, 706 of the detection chambers. The entire carrier is insertedinto the detection chamber 701, and the cover 80 is clamped on the panel 13 and also seals the opening of the detection chamber. Generally, the cover is made of rubber, which is convenient for assembly during production. The cover 80 is provided with a channel 81 communicating with an external atmosphere to reduce generation of gas pressure or emission of compressed air caused by a fact that the collector enters the sample chamber to compress air, thereby smoothly allowing the liquid in the sample chamber to enter the bottom 706 of the detection chamber. The detection chamber 70 and the sample chamber may be integrally formed, such that the liquid can be released when the collector is inserted into the sample chamber, and the bottom of the sample chamber is in communication with the bottom of the detection chamber, and the liquid released by the collector can normally flow to the bottom of the detection chamber and contact the end portion of the sample application area located at the bottom of the detection chamber to perform the primary test. When the detection chamber and the sample chamber are assembled, the sample chamber integrated with the detection chamber 703 is provided. One end of the detection chamber is provided with an opening 701 and the other end thereof is sealed, and the sample chamber is in communication with the bottom of the detection chamber. Then, the carrier 13 is provided, and the testing element is loaded in the groove of the carrier, and the end portion of the sample application area of the carrier is proximal to the bottom of the detection chamber, such that the liquid entering the bottom of the detection chamber contacts the sample application area to complete the primary test of the analyte in the sample. One liquid channel 125 is also provided between the sample chamber and the detection chamber. The insertion of the collector facilitates the liquid collected from the sample chamber to flow into the detection chamber through the liquid channel 125 and contact the testing element to complete the primary test. If the secondary test needs to be performed, a plug705 is removed (the plug seals a hole 704), and the liquid is directly absorbed from the hole 704 in the bottom of the detection chamber through an absorption tube to perform the secondary validation test. This can be realized when the secondary test is expected to be performed immediately after the primary test, with convenient and fast sampling. The sample chamber includes a limit structure 13, where the limit structure has an opening 132 and an insertion element 131, and the insertion element is inserted into an insertion port at the periphery of the opening of the sample chamber and can also be fixed together with threads of the collector. When the test needs to be performed, the entire collector and the limit structure together collect the liquid sample, and then the liquid sample is inserted into the sample chamber; in this case, the absorption element is allowed to be compressed to release the liquid sample; after the liquid sampleis completely released, the limit structure can be disassembled from the collector, and then the collector is allowed to be inserted into the sample chamber in a deeper depth, such that the absorption element can be completely compressed. The collector and the sample being in a sealed state may facilitate more liquid to enter the detection chamber. In the embodiment, the base 11 has a groove 111 for placing the test detection chamber, and a recess 112 for placing the bottom of the sample chamber.

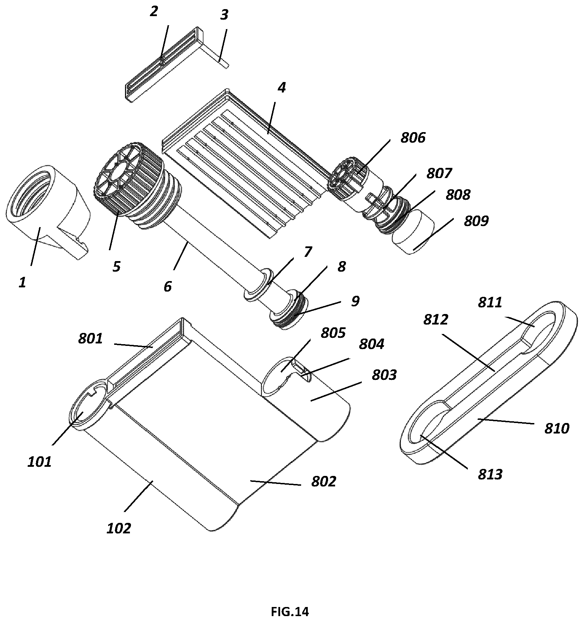

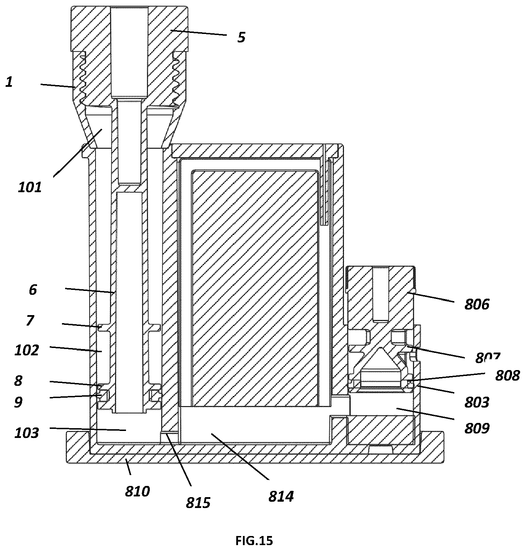

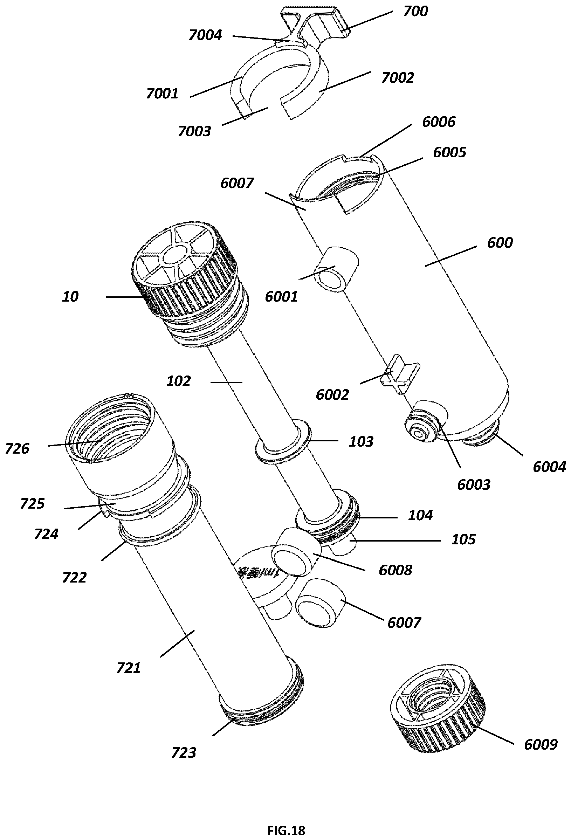

FIG. 14-FIG.18 show another specific embodiment of the present invention. In the specific embodiment, the sample chamber and the detection chamber are of an integral structure, while the secondary validation test chamber and the sample chamber are respectively in fluid communication with the detection chamber, and the secondary validation test chamber is located on a side of the detection chamber instead of being in the sample chamber. As shown in FIG. 14, the specific embodiment provides a detection chamber 802, a sample chamber 102 and a secondary validation test chamber 803. The sample chamber 102, the detection chamber 802 and the secondary validation test chamber 803 are molded through injection at one time, and cannot be separated or combined in a detachable manner. Specifically, the sample chamber is located at a side of the detection chamber 802, and the secondary validation test chamber 803 is located at another side of the detection chamber 802. The detection chamber includes a carrier 4, and a plurality of grooves are arranged on the carrier for accommodating the testing element for the primary test, such as a lateral flow element. The detection chamber has an opening 801 for inserting the carrier with the testing element into the detection chamber, and then a sealing cover 2 is used to seal the opening of the detection chamber, and the sealing cover 2 is provided with a pipe 3 communicating with the external atmosphere, to keep the inside of the detection chamber be in fluid communication with the external atmosphere. Here, the secondary validation test chamber 803 itself is not used for secondary validation, but a second collector is inserted into the secondary validation test chamber 803; the second collector has a second absorption element 809, two sealing elements 808 connected to the collector, a first sealing element 807, and a hand-held portion 806, and left and right sides of the sealing element are in sealing fit with an inner wall of the secondary validation test chamber 803. Thus, the second collector is inserted into the secondary validation test chamber 803, such that the second absorption element 809 is located at the bottom of the secondary validation test chamber 803, and the bottom of the secondary validation test chamber 803 is in fluid communication with the bottom of the detection chamber 802 (as shown in FIG. 15). The collector is just as the collectors in the above-mentioned specific embodiments, the rod-shaped end portion of the collector is provided with a sealing element 8, and a first absorption element 9 for absorbing the liquid sample is arranged below the sealing element. In the embodiment, the collector for collecting the liquid sample may be called the first collector, and the first collector is a collector used for collecting the liquid sample for the first time and performing the primary test. Referring to FIG. 14 and FIG. 15, the secondary validation test chamber 803 has an opening 805 and a small notch 804. The opening is used to allow the second collector to be inserted into the secondary validation test chamber 803 and is sealed. In addition, the first sealing element 807 and the second sealing element 808 on the second collector fit with the inner wall of the secondary validation test chamber 803 to form hermetic seal, and this is mainly to prevent the liquid absorbed by the second absorption element 803 from volatilization due to the change of an external temperature. "Volatilization" herein mainly means that the liquid absorbed by the absorption element evaporates due to the change of external environmental conditions, such as temperature change (for example, temperature increase), and thus the liquid cannot be retained. In order to allow the second collector to be more firmly fixed together with the secondary validation test chamber 803, the collector includes a protrusion 815, where the protrusion fits with the notch 804 on the secondary validation test chamber. Referring to a cross-section diagram shown in FIG. 15, at an initial position, a first collector is not provided in the sample chamber 102, but a second collector is provided in the secondary validation test chamber. When the test is performed, the first absorption element 9 of the first collector 5 is used to collect the liquid sample, and then is inserted into the sample chamber 102 from the opening 101 thereof. At the bottom of the sample chamber, the first absorption element is compressed to release the liquid sample, and the released liquid sample flows to the bottom 814 of the detection chamber 802 through the liquid channel 805, a part of the released liquid sample contacts the testing element on the carrier in the detection chamber 802 for the primary test, and the other part of the released liquid sample flows into the secondary validation test chamber and is absorbed by the second absorption element 809 on the second collector and stored in the second absorption element 809. Of course, the so-called part refers to the liquid absorbed by the testing element, while the liquid not absorbed by the testing element can be absorbed by the second absorption element 809. It can be understood that the volume of the liquid sample compressed in the first collector is greater than the saturated absorption capacity of the second absorption element. For example, the volume of the liquid samplereleased by compressing the first absorption element on the first collector is 2 ml, about 1 ml of the liquid sample is used for the primary test, and the other 1 ml or 500 µL of the liquid sample is absorbed by the second absorption element of the second collector and stored in the second absorption element 809. When the secondary test needs to be performed, the second collector is directly removed from the secondary validation test chamber and independently packaged in a container similar to a small bottle, and the container is delivered to the laboratory for secondary validation test. When the array is performed, the second absorption element 809 is allowed to be compressed, thereby releasing the liquid for secondary validation test. As mentioned above, when the absorption element is used to store the liquid, and even if an amount of liquid is small, the secondary test can still be completed; especially when an amount of the liquid to be tested is small, such as saliva samples, an amount of collected saliva samples is relatively small and saliva is sticky, so the absorption element is used to absorb the saliva samples, which can prevent the saliva samples from evaporating and drying. If the saliva is not collected by a chamber without an absorption element and used for secondary validation, it may evaporate and dry due to absence of an absorption carrier, such that the secondary validation test cannot be performed well and conveniently. If a solution is added to dissolve the dried sample, the concentration of the dried sample will be diluted, and the dried sample may not be completely dissolved, thereby resulting in inaccurate secondary test results.

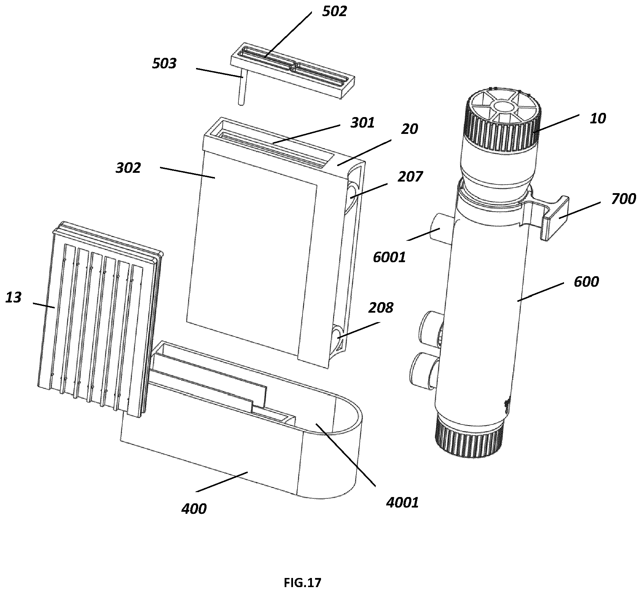

FIG. 16-FIG. 19 show another specific embodiment of the present invention. In the embodiment, the sample chamber includes a movable chamber, and the collector is inserted into the movable chamber. When the movable chamber is located at the first position, the collector is inserted into the movable chamber to compress and release the liquid sample on the first collector. A part of the released liquid flows to the detection chamber to complete the primary test, while the other part thereof flows to the second absorption element in the sample chamber to be absorbed and preserved. When the secondary test needs to be performed, the movable chamber is allowed to be moved downward to compress the second absorption element to release the stored liquid sample for the second validation test. FIG. 16 shows an assembled test device according to the specific embodiment of the embodiment. Just as the above-mentioned test device, the assembled test device includes a detection chamber 30 and a sample chamber 600, where the detection chamber is in fluid communication with the bottom of the sample chamber through a liquid channel 780. The detection chamber includes a carrier 13 including a plurality of grooves 1303, where each of the grooves is provided with one testing element, for example, a lateral flow testing element (FIG. 20); the carrier is inserted into the detection chamber, such that the end portion of the sample application area 405 of the testing element is proximal to the bottom 785 of the detection chamber, and the opening 301 of the detection chamber is sealed by the sealing cover 502. Similarly, the sealing cover is provided with a pipe 503 to allow the detection chamber to be kept in gas communication with the outside, such that gas pressure in the detection chamber and the external atmospheric pressure are consistent or equal. A socket 208 is arranged on a side wall of the detection chamber 30 and connected with a pin 6003 of the sample chamber; the pin has a liquid channel 780, such that the sample chamber 600 is kept in fluid communication with the detection chamber 30 through the liquid channel. For example, as shown in FIG. 17, the detection chamber may further include a socket 207 that is recessed, and the socket is arranged on the wall of the detection chamber 30. Moreover, the sample chamber 600 is provided with an insertion element 6001, and the insertion element can be inserted into the socket 207. On the one hand, the sample chamber 600 is allowed to be detachably connected with the detection chamber 30; on the other hand, one liquid channel 780 is provided to communicate the sample chamber 600 with the detection chamber 30 (FIG. 19). The socket 208 is proximal to the bottom of the detection chamber, while the socket 207 is proximal to the opening thereof. Except for a fitting mode of the socket and the pin, any other detachable modes can be applied to the present invention, such as lock, button, hinge and plug-in mode, and the liquid channel is arranged only in a specific detachable structure to allow the sample chamber to be in fluid communication with the detection chamber. According to the present invention, the socket 208 and the pin 6003 are detachably connected, and the liquid channel is provided to allow the sample chamber to be in fluid communication with the detection chamber. When the sample chamber and the detection chamber need to be separated, the pin 6003 of the sample chamber is allowed to be detached from a jack 208, or the insertion element 6001 is allowed to be detached from the jack 207 to realize the separation thereof.

In some embodiments, the sample chamber 600 can further be provided with a protrusion 6002; the protrusion is not connected with the detection chamber, but is provided with a sealing cover 6008; the sealing cover seals the liquid channel 780 in the sample chamber when the sample chamber 600 and the detection chamber are separated. In some embodiments, the movable chamber 721 is located in the sample chamber 600, and the bottom 782 of the movable chamber is proximal to the bottom of the sample chamber; a space is arranged between the bottom of the movable chamber and the bottom of the sample chamber, and the space is the secondary validation test chamber and used for receiving the liquid sample from the collector. A part of the liquid sample is absorbed by the second absorption element 716 located in the space, while the other part of the liquid sample flows into the detection chamber through the liquid channel for the primary assay.

In some embodiments, the movable chamber 721 includes an opening 426 for receiving the collector 10. In addition, a first sealing element 722 and a second sealing element 723 are arranged on the outer wall of the chamber, and such sealing elements fit with the inner wall 6010 of the sample chamber to form hermetic seal. The second sealing element 723 is arranged close to the bottom of the movable chamber, such that the bottom of the movable chamber and the bottom of the sample chamber form a hermetic seal structure. In some embodiments, the bottom of the movable chamber has a hole 784; when the collector is inserted into the hole, the first absorption element 105 contacts the bottom of the movable chamber and interacts with the bottom of the movable chamber, the first absorption element is compressed to release the liquid, and the released liquid flows into the secondary validation test chamber below the first absorption element through the hole 784. In some embodiments, the movable chamber has a first position and a second position in the sample chamber, the first position and the second position are controlled by the limit structure. When the limit structure is provided, the movable chamber is located at the first position; and if the limit structure is removed, the movable chamber can move from the first position to the second position. In some embodiments, the limit structure is a snap ring 700 with an annular structure, and the annular structure includes a notch 7003; the notch is laterally clamped outside the movable chamber, and a first fixed position of the movable chamber is limited by the opening of the sample chamber. In order to allow the position of the movable chamber to be fixed, an annular structure 724 is arranged near the outer wall of the movable chamber proximal to the opening, thus forming a snap ring area 725; and the snap ring is clamped in the snap ring area, such that the movable area can be better fixed at the first position. In order to allow the position of the snap ring to fit with the inlet of the sample chamber, an interface 6006 and an edge protruding area 6007 at the inlet of the sample chamber. The area and shape of the edge protruding area are just the radian and height of the notch 7003 of the snap ring, and the interface 6006 is an area where the hand-held area 7005 of the snap ring is allowed to be clamped. Thus, the snap ring is fixedly clamped on the outer wall of the movable chamber, and fixed to the sample chamber, and does not rotate axially. As shown in FIG. 19 and FIG. 16, the movable chamber 721 is at an initial position. In this case, the chamber formed by the bottom 782 of the movable chamber and the bottom of the sample chamber is the secondary validation test chamber, and the secondary validation test chamber includes the second absorption element 716. Just as the above-mentioned embodiment, the secondary validation test chamber actually has two holes communicating with the outside, one hole is the liquid channel 780, and the other hole is a hole 6004 in the bottom of the sample chamber, but the hole is not sealed with the cover 6009. In addition, the secondary validation test chamber is not completely filled with the second absorption element, but partially filled with it, and a space 781 is reserved between the bottom of the movable chamber and the upper end of the second absorption element. As shown in FIG. 19, when the test is performed, the collector is used to collect the liquid sample, for example, the first absorption element 105 of the collector is used to collect the liquid sample, and is allowed to absorb the liquid sample to a saturated state or fully absorb the liquid sample, and then the collector is inserted into the movable chamber; thus sealing elements 103, 104 on the collector 10 and the inner wall of the movable chamber are hermetically sealed, the first absorption element is compressed to release the liquid sample, and the liquid sample almost entirely flows into the secondary validation test chamber below the first absorption element through the hole 784 in the bottom 782 of the movable chamber. This is because the collector needs to be inserted into the movable chamber and form a sealing structure with the inner wall of the movable chamber, and also needs to move in the movable chamber, the first absorption element on the collector is allowed to contact the bottom 782 of the movable chamber and is compressed. In fact, when the collector is inserted into the movable chamber and moves, it needs to compress air in the space in the movable chamber to reduce its volume, thereby increasing pressure of gas. The so-called space here refers to the space sealed by the collector in the movable chamber and also includes the space for secondary validation of samples. In addition, the first absorption element is compressed to release the liquid. In order to allow the liquid to flow smoothly into the secondary validation test chamber, a hole 784 is opened in the bottom of the movable sample chamber, and the released liquid quickly flows into the secondary validation test chamber through the hole 784 under the pressure. In some embodiments, the secondary validation test chamber 782 includes the second absorption element. After the liquid released from the first absorption element is absorbed by the second absorption element to a saturated state, the excess liquid is located in the space 782; the increasing pressure in the secondary validation test chamber 782 facilitates the excess liquid to flow into the bottom 785 of the detection chamber 705 through the liquid channel 780, and the excess liquid contacts the testing element at the bottom of the detection chamber to complete the primary test of the liquid sample.

When the primary test of the liquid sample is completed, the insertion elements 6001, 6003 of the sample chamber are detached from the jacks 207, 208 of the detection chamber, such that the sample chamber and the detection chamber are separated, and the cover 6008 is disassembled from a protruding structure 6002 and sealed on the insertion element that includesthe liquid channel 780, thus sealing the entire secondary validation test chamber. In fact, the collector is used tohermetically seal the inside of the movable chamber and the movable chamber is used to hermetically sealthe inside of the sample chamber; in the formed hermetic seal space, only the liquid channel is in communication with the detection chamber, while the detection chamber is in communication with the external atmosphere through a pipe 707. Therefore, as the collector moves, the volume of the formed hermetic seal space decreases and the pressure thereof increases, and the increased pressure facilitates the excess liquid (which refers to the remaining liquid sample after the released liquid is absorbed by the second absorption element 716 to a saturated state) to flow into the bottom of the detection chamber only through the liquid channel 780. It can be understood that inserting the collector into the movable chamber, sealing the inside of the movable chamber, and compressing the first absorption element to release the liquid do not need to take a long time, and are substantially completed in a few seconds or dozens of seconds. In some embodiments, a distance is allowed to exist between the surface of the second absorption element and the bottom of the movable chamber, and is not occupied by the second absorption element, as shown in FIG. 22. Thus, after the second absorption element absorbs the liquid sample to a saturated state, the excess liquid flows into the space, and the space is in fluid communication with the liquid channel 780, thereby increasing pressure with the movement of the collector. Such pressure facilitates the liquid in the space to flow into the detection chamber through the liquid channel 780 for the primary test or the first test.

When the primary test is completed, the sample chamber 600 and the detection chamber can be separated, and the sample chamber includes the movable chamber 721, the first collector 10, and the second absorption element located in the sample chamber 600; in this case, the cover 6008 is used to seal the insertion element 6003 including the channel, and the entire secondary validation test chamber is in a sealed state and can be transported to the laboratory for the secondary validation test. When the secondary validation test needs to be performed, it can be implemented by the following ways. In a first method, the cover 6008 is removed to allow the liquid channel 780 to be in communication with the external atmosphere, and the snap ring 700 is removed to allow the movable chamber to continue to move in the sample chamber to apply the pressure to the second absorption element 716, and the second absorption element is compressed to release the liquid by applying the pressure thereon; and when the movable chamber moves downward, the pressure of the compressed air increases, and such pressure facilitates the liquid released from the second absorption element to flow out of the liquid channel 780 (for example, a test tube) for the secondary validation test. In a second way, the cover 6008 is not removed, but the cover 6009 at the bottom of the sample chamber is removed; then, the second absorption element is compressed to release the liquid, and the released liquid flows out of the bottom of the sample chamber for the secondary validation test. It can be understood that one hole 6004 is provided at the bottom of the sample chamber 600 and is sealed by the cover 6009, and the second absorption element is T-shaped and fixed to a T-shaped step; thus, when the movable chamber 721 moves downwards, the bottom thereof directly contacts the second absorption element and compresses it, and the excess liquid flows out of an opening 786 through the lower part 788 of the second absorption element. In some embodiments, in order to allow the first way to be more easily implemented, a protruding structure 789 is provided at the hole 784 in the bottom of the movable chamber; and the protruding structure extends from the bottom to the surface against the second absorbent element. With the downward movement of the movable chamber 721, the top of the extension structure contacts the second absorbent element, the extension structure compresses the second absorption element to release the liquid, the released liquid flows into the space between the surface of the second absorption element and the bottom of the movable chamber, and the liquid channel 780 communicating the outside is provided in the space; thus, the liquid flowing to the space flows to the outside through the liquid channel 780 for the secondary validation test. Of course, the extension structure can also be some protrusions extending from the bottom of the movable chamber toward the surface of the second absorption element, and the protrusions can also apply pressure to the second absorption element, thereby compressing the second absorption element to release the liquid sample for secondary validation.

### Embodiment 1 Effects of presence or absence of absorption elements on storage of liquid samples according to the present invention

Referring to examples as shown in FIG. 4 and FIG. 5, the sample chamber 60 is provided, the collector is used to absorb a saliva sample, the absorption element 70 of the collector absorbs 2 ml of the saliva sample to a saturated state, and then is inserted into the sample chamber. The absorption element is compressed, such that a part of the saliva sample is absorbed by the second absorption element 70 (the saliva sample absorbed by the second absorption element to a saturated state is 0.8 ml), the excess saliva sample flows out of the liquid channel 607, and the volume of the saliva sample flowing out of the liquid channel is 1.0 ml. After the excess saliva sample flows out of the liquid channel, the liquid channel is sealed with the cover 204.

Correspondingly, a same structure is used for the sample chamber regardless of presence or absence of absorption elements, except that there is no second absorption element and 0.8 ml of the saliva sample is kept in the space 201 without the second absorption element. In other words, a same volume of the saliva sample is absorbed and preserved by the second absorption element of the present invention, or not absorbed and preserved by the second absorption element but preserved in the chamber, and the second absorption element is made of polyester foam.

The liquid sample storage capacity of the sample chamber in different states is investigated. The specific conditions are given below: the above sample chambers each store five liquid samples, the average value thereof is calculated, and the sample chambers are respectively placed in different sealing containers, and each sealing container is stored with five sample chambers. The sealing containers are connected with a negative pressure compression device, air therein is pumped out to keep them be in a constant negative pressure state for different times, and the remaining saliva samples in different conditions are measured. For the sample chamber excluding the second absorption element, the remaining saliva sample is directly measured; for the sample chamber including the absorption element, the absorption element is compressed and the volume of the released liquid is observed. Specific results are as shown in Table 1:

**Table 1: Storage capacity of saliva samples in sealing containers at ambient temperature under negative pressure**

| Conditions | Presence of second absorption element | Absence of second absorption element |
|---|---|---|
| Saliva samples are stored for 24 hours in the sealing containers at ambient temperature under negative pressure | 0.75 ml | 0.79 ml |
| Saliva samples are stored for 48 hours in the sealing containers at ambient temperature under negative pressure | 0.74 ml | 0.75 ml |
| Saliva samples are stored for 72 hours in the sealing containers at ambient temperature under negative pressure | 0.73 ml | 0.65 ml |
| Saliva samples are stored for 120 hours in the sealing containers at ambient temperature under negative pressure | 0.70 ml | 0.45 ml |
| Saliva samples are stored for 240 hours in the sealing containers at ambient temperature under negative pressure | 0.68 ml | 0.30 ml |
| Saliva samples are stored for 240 hours in the sealing containers at ambient temperature under negative pressure | 0.65 ml | 0.15 ml |

It can be seen from Table 1 that under such negative pressure, the sample chamber including the absorption element can continuously maintain the liquid sample storage capacity, but still has slight decrease mainly with negative pressure and evaporation, and the liquid sample storage capacity of the sample chamber excluding the absorption element rapidly decreases. The reason for evaporation is that it is impossible to accurately seal the sealing containers, but evaporation can occur under negative pressure, resulting in reduction in the liquid volume. The negative pressure is to mainly simulate transportation environment under air transportation.

**Table 2: Storage capacity of liquid in sealing containers at a temperature of 45°C and a humidity of 50% under negative pressure**

| Conditions | Presence of second absorption element | Absence of second absorption element |
|---|---|---|
| Liquid is stored for 24 hours in the sealing containers at a temperature of 45°C and a humidity of 50% under negative pressure | 0.73 ml | 0.70 ml |
| Liquid is stored for 48 hours in the sealing containers at a temperature of 45°C and a humidity of 50% under negative pressure | 0.70 ml | 0.65 ml |
| Liquid is stored for 72 hours in the sealing containers at a temperature of 45°C and a humidity of 50% under negative pressure | 0.68 ml | 0.48 ml |
| Liquid is stored for 96 hours in the sealing containers at a temperature of 45°C and a humidity of 50% under negative pressure | 0.65 ml | 0.35 ml |
| Liquid is stored for 120 hours in the sealing containers at a temperature of 45°C and a humidity of 50% under negative pressure | 0.64 ml | 0.15 ml |
| Liquid is stored for 240 hours in the sealing containers at a temperature of 45°C and a humidity of 50% under negative pressure | 0.60 ml | 0.05 ml |

It can be seen from results in Table 2 that under conditions of constant negative pressure, relatively high temperature and relatively dry external environment, the liquid sample storage capacity of the sample chamber including the absorption element is relatively excellent; generally, the secondary validation test chambers are transported to the laboratory by air or freight from a place where the primary test is performed. During the air transportation, it is easy to reduce volatilization of the liquid in a negative pressure and dry environment, and even sometimes secondary validation cannot be performed because the liquid samples evaporate and dry; in the present invention, the absorption element is used to store the liquid sample, which can effectively avoid the evaporation of liquid; therefore, the absorption element can be used for secondary validation.

All the patents and publications mentioned in the description of the present invention indicate that these are public technologies in the art and can be used by the present invention. All the patents and publications cited herein are listed in the references, just as each publication is specifically referenced separately. The present invention described herein can be realized in the absence of any one element or multiple elements, one restriction or multiple restrictions, where such restriction is not specifically described here. For example, the terms "comprising", "essentially consisting of' and "consisting of' in each embodiment herein may be replaced by the rest 2 terms. The so-called "alan" herein merely means "one", but does not exclude including 2 or more instead of including only one. The terms and expressions which have been employed herein are descriptive rather than restrictive, and there is no intention to suggest that these terms and expressions in this description exclude any equivalents, but it is to be understood that any appropriate changes or modifications can be made within the scope of the present invention and appended claims. It can be understood that the embodiments described in the present invention are some preferred embodiments and features. A person skilled in the art can make some modifications and changes according to the essence of the description of the present invention. These modifications and changes are also considered to fall within the scope of the present invention and the scope limited by independent claims and dependent claims.

## Claims

1. A device for detecting an analyte in a liquid sample, comprising:
a sample chamber for accommodating a collector; and
a detection chamber in fluid communication with the sample chamber;
wherein the detection chamber comprises a testing element therein, and the testing element is configured to detect the analyte in the liquid sample for the first time;
and the sample chamber is detachably combined with the detection chamber, and the collector comprises a first absorption element that is used for absorbing the liquid sample and is capable to be compressed;
a secondary validation test chamber, wherein the secondary validation test chamber comprises a second absorption element that is capable to absorb the liquid sample and be compressed.

2. The device according to claim 1, wherein when the sample chamber comprises the collector, the first absorption element of the collector is capable to be compressed to release the liquid sample, wherein a part of the released liquid sample is capable to flow into the secondary validation test chamber and be absorbed by the second absorption element.

3. The device according to one of claims 1-2, wherein the secondary validation test chamber is in fluid communication with the detection chamber, and the liquid sample absorbed by the second absorption element is used for secondary validation test.

4. The device according to one of claims 1-3, wherein a volume of liquid sample absorbed by the second absorption element in the secondary validation test chamber to a saturated state is constant.

5. The device according to one of claims 1-4, wherein a volume of the liquid sample released by compressing the first absorption element is greater than or equal to the volume of liquid sample absorbed by the second absorption element to a saturated state.

6. The device according to one of claims 1-5, wherein the secondary validation test chamber is located in the sample chamber.

7. The device according to one of claims 1-6, wherein the secondary validation test chamber is in fluid communication with the detection chamber through a liquid channel.

8. The device according to claim 7, wherein the secondary validation test chamber is in fluid communication with the sample chamber through the detection chamber and the liquid channel.

9. The device according to claim 8, wherein the second absorption element is arranged on an absorber with a handle and the absorber is capable to depart from the secondary validation test chamber.

10. The device according to one of claims 1-9, wherein the sample chamber comprises a first protrusion that comprises the liquid channel, and the first protrusion is detachably connected with a first recessed interface of the detection chamber, such that the sample chamber is capable to be in detachable communication with the detection chamber through the liquid channel.

11. The device according to one of claims 1-10, wherein the sample chamber further comprises a cover, and the cover is configured to seal the liquid channel when the sample chamber and the detection chamber are separated.

12. The device according to claim 11, wherein when the sample chamber comprises the collector, the sample chamber is sealed by the collector, such that the secondary validation test chamber is hermetically sealed; in this way, if the secondary validation test chamber is capable to be compressed by moving the collector, gas in the secondary validation test chamber is allowed to be compressed to increase pressure therein.

13. The device according to one of claims 1-12, wherein the sample chamber further comprises a movable chamber, the movable chamber is configured to receive the collector and allow the first absorption element to be compressed to release the liquid sample, and the movable chamber has a first position and a second position in the sample chamber.

14. The device according to one of claims 1-12, wherein when the absorption element is compressed to release the liquid, the released liquid flows to the secondary validation test chamber, the second absorption element is allowed to absorb the liquid, and the excess liquid flows to the detection chamber through the liquid channel to contact the testing element for testing and assaying, and the movable chamber is located at the first position.

15. The device according to claim 14, wherein after the collector and the detection chamber are separated, and when secondary validation test needs to be performed, the movable chamber and the collector are allowed to move together to the second position, and the second absorption element is compressed by the movable chamber, whereby releasing the liquid through the liquid channel; and the released liquid is used for secondary validation test.
